(19) Europäisches Patentamt
European Patent Office
Office européen des brevets

(11) Numéro de publication : **0 435 794 A1**

(12) **DEMANDE DE BREVET EUROPEEN**

(21) Numéro de dépôt : **90420573.9**

(22) Date de dépôt : **27.12.90**

(51) Int. Cl.[5] : **C07D 307/38,** C07D 307/64, C07D 333/18, C07D 333/34, C07D 207/333, C07D 207/36, C07D 263/32, C07D 263/46, C07D 261/10, C07D 275/03, // C07D277/26, C07D277/36, C07C317/12, C07C317/18, C07C317/22, C07D401/12, C07D405/12, C07D409/12, C07D413/12, C07D417/12, C07D405/14, C07D409/14, C07F7/08

(30) Priorité : **28.12.89 FR 8917544**

(43) Date de publication de la demande : **03.07.91 Bulletin 91/27**

(84) Etats contractants désignés : **AT BE CH DE DK ES FR GB GR IT LI LU NL SE**

(71) Demandeur : **RHONE-POULENC AGROCHIMIE**
**14-20, rue Pierre Baizet**
**F-69009 Lyon (FR)**

(72) Inventeur : **Guigues, François**
**936, avenue Victor Hugo**
**F-69140 Rilleux (FR)**
Inventeur : **Bascou, Jean-Philippe**
**5, chemin de Monpellas**
**F-69009 Lyon (FR)**
Inventeur : **Pearson, Christopher John,**
**Research Station of**
**Rhône-Poulenc Agriculture, Fyfield Road**
**Ongar, Essex CM5 0HW (GB)**
Inventeur : **de Reinach Hirtzbach, François**
**5, quai Général Sarrail**
**F-69006 Lyon (FR)**
Inventeur : **Desbordes, Philippe**
**30, rue Bancel**
**F-69007 Lyon (FR)**
Inventeur : **Euvrard, Michel**
**33, route du Mont Thou, Saint Romain au Mont d'Or**
**F-69270 Fontaines sur Saone (FR)**

(74) Mandataire : **Brachotte, Charles et al**
**RHONE-POULENC AGROCHIMIE P.I.D. BP 9163**
**F-69263 Lyon Cedex 09 (FR)**

(54) **Herbicides à groupe alcenyle ou hétéroaryle thio, sulfone, sulfoxyde.**

(57) Alcényl ou hétéroaryl sulfones herbicides
Herbicides de formule :

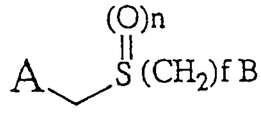

(I)

dans laquelle :
n = 0, 1, 2
f = 0, 1
A est choisi parmi les groupes

**EP 0 435 794 A1**

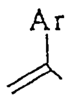 $(A_1)$ 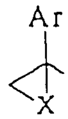 $(A_2)$ 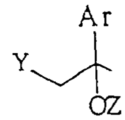 $(A_3)$ 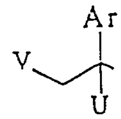 $(A_4)$

dans lesquels :

Ar est choisi parmi les groupes

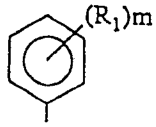 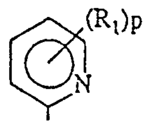 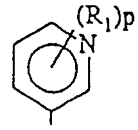 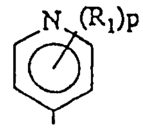

Ar-1 Ar-2 Ar-3 Ar-4

B est choisi parmi les groupes $C_2$ - $C_{10}$ alcényle éventuellement substitués ou choisi parmi les groupes thiophène, furanne, pyrrole, thiazole, isothiazole, oxazole ou isoxazole éventuellement substitués.

X étant oxygène ou soufre,

Y étant halogène ou sulfonate,

Z étant hydrogène ou ester,

U et V étant halogènes.

# HERBICIDES A GROUPE ALCENYLE OU HETEROARYLE THIO, SULFONE, SULFOXYDE

L'invention est relative à de nouveaux composés, à leur utilisation à titre d'herbicides notamment sous forme de composition herbicide, à un procédé de contrôle des mauvaises herbes à l'aide de ces composés ou de ces compositions.

Un objet de la présente invention est donc de proposer des composés utiles en pré ou post émergence comme herbicides.

Un autre objet de la présente invention est de proposer des composés utiles en pré ou post émergence comme herbicides antigraminées.

Un autre objet de la présente invention est de proposer des composés utiles en pré ou post émergence comme herbicides sélectifs du maïs et de nombreuses cultures dicotylédones (notamment soja, colza, tournesol, coton) et autres monocotylédones (blé, riz).

Définition générale de l'invention :

Composés de formule :

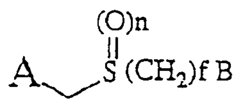

(I)

dans laquelle :

n = 0, 1, 2

f = 0, 1

A est choisi parmi les groupes

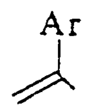 ($A_1$) 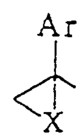 ($A_2$)

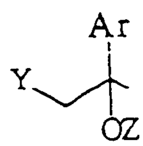 ($A_3$) 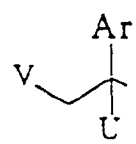 ($A_4$)

dans lesquels :

Ar est choisi parmi les groupes

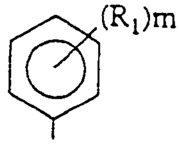 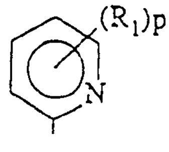 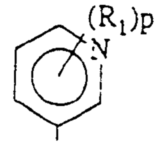 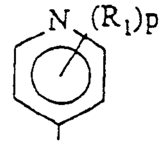

Ar-1 Ar-2 Ar-3 Ar-4

X étant un atome d'oxygène ou de soufre,

$R_1$ étant un atome d'halogène (notamment Cl ou Br ou F), un groupe $C_1$-$C_4$ alkyle, $C_1$-$C_4$ alkoxy, $C_1$-$C_4$ haloalkyle, $C_1$-$C_4$ haloalkoxy, nitro, cyano, $C_6$-$C_{10}$ aryle (notamment phényle ou naphtyle), $C_7$-$C_{11}$ aralkyle (notamment benzyle), $C_6$-$C_{10}$ aryloxy (notamment phénoxy ou naphthoxy) éventuellement substitué par 1 ou 2 atomes d'halogène, $C_7$-$C_{11}$ aralkyloxy (notamment benzyloxy) éventuellement

substitué par 1 ou 2 atomes d'halogène,
m = 0, 1, 2, 3, 4, 5,
p = 0, 1, 2, 3, 4,
les différents radicaux $R_1$ étant identiques ou différents lorsque m ou p est supérieur ou égal à 2,
Y est un atome de chlore ou de brome ou d'iode ou un groupement $OSO_2R_2$,
$R_2$ étant un groupement $C_1$-$C_6$ alkyle, $C_6$-$C_{10}$ aryle, $C_7$-$C_{11}$ aralkyle, les dits groupements étant éventuellement substitués par 1 à 3 halogènes ou groupements $C_1$-$C_4$ alkyle, $C_1$-$C_4$ alkoxy, $C_1$-$C_4$ haloalkyle, $C_1$-$C_4$ haloalkoxy, nitro,
Z étant un atome d'hydrogène ou un groupe
(C=O)$R_5$,
$R_5$ est hydrogène, $C_1$-$C_4$ alkyle,
$C_1$-$C_4$ haloalkyle,
U étant un atome de brome ou de chlore,
V étant un atome de chlore, de brome ou d'iode,
B est choisi parmi les groupes $C_2$-$C_{10}$ alcényle, ces groupes étant éventuellement substitués par 1 à 6 atomes d'halogène ou choisi parmi les groupes

B1 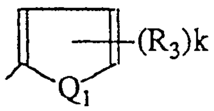 B2 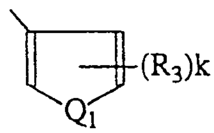

B3 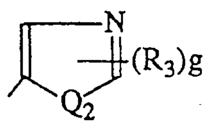 B4 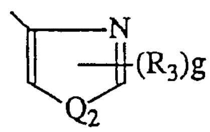 B5 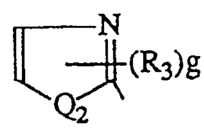

B6 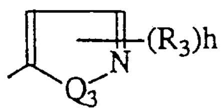 B7 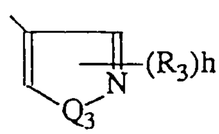 B8 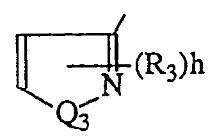

$R_3$ ayant l'une des significations indiquées pour $R_1$ ou le groupe $C_1$-$C_4$ thioalkyl, les différents radicaux $R_3$ étant identiques ou différents lorsque k ou g ou h est supérieur ou égal à 2,
$Q_1$ étant un atome de soufre, d'oxygène ou le groupe $NR_4$,
$Q_2$ étant un atome de soufre ou d'oxygène,
$Q_3$ étant un atome de soufre ou d'oxygène,
$R_4$ est hydrogène, $C_1$-$C_4$ alkyle, $C_1$-$C_4$ haloalkyle,
k = 0, 1, 2, 3,
g = 0, 1, 2,
h = 0, 1, 2.

Variantes préférées :

Selon des modes de réalisation préférés, on choisira les variantes suivantes prises ou non en combinaison:
- n= 2
- X = 0
- Z = H
- U,V = Br
- m inférieur ou égal à 2
- p inférieur ou égal à 2
- k inférieur ou égal à 2
- $R_1$ est halogène, nitro, trifluorométhyle, méthoxy, méthyle, éthyle.

Les composés de formule (I) et les composés éventuellement utilisables à titre d'intermédiaires dans les

procédés de préparation, et qui seront définis à l'occasion de la description de ces procédés, peuvent exister sous une ou plusieurs formes d'isomères selon le nombre de centres asymétriques de la molécule. L'invention concerne donc aussi bien tous les isomères optiques que leurs mélanges racémiques et les diastéréoisomères correspondants. La séparation des diastéréoisomères et/ou des isomères optiques peut s'effectuer selon les méthodes connues en soi.

Procédé de préparation

Méthode A

Les composés de formule (I) pour lesquels n = 2, et A est (A1), les autres substituants ayant la même définition que celle indiquée dans la définition générale de l'invention peuvent être obtenus par mise en contact d'un composé de formule :

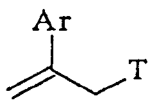

(II)

dans laquelle Ar, $R_1$ et m ou p ont la même signification que dans la définition générale de l'invention,
T est un atome de chlore ou de brome, avec un composé de formule :

$MSO_2$ $(CH_2)_f$ - B (III)

f, B ayant la même définition que celle indiquée dans la définition de l'invention,
M étant un atome de métal alcalin ou alcalino-terreux (notamment Li, K, Na).

La réaction est généralement effectuée dans un solvant aprotique dipolaire notamment le diméthylformamide, la N-méthyl pyrrolidone, ou dans l'eau en mélange dans les proportions 5/95 à 90/10 (de préférence 10/90 à 50/50) avec un solvant soluble dans l'eau comme les alcools, l'acétone, l'acétonitrile le diméthoxyéthane, en présence ou non, d'une quantité catalytique ou non d'iodure alcalin, à une température comprise entre 25°C et 150°C (de préférence 60°C à 120°C) et dans un rapport molaire II :III compris entre 1 et 10 (de préférence 1 et 2).

Cette réaction est notamment connue par "J. March, Advanced Organic Chemistry" Ed. Mc Graw-Hill (1985) p. 363.

Les composés de formule (II) où T est chlore, et Ar est Ar-1 (noyau phényle) sont préparés par chloration d'un composé 2-phényl 1-propène de formule :

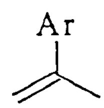

(IV)

dans laquelle Ar est Ar-1 (noyau phényle),
$R_1$ et m ayant la même signification que dans la définition de la formule générale, au moyen du réactif $Ca(OCl)_2/CO_2$. Cette réaction est décrite par S.G. Hegde et J. Wolinsky Tetrahedron Letters (1981), 22, 5019.

Ces mêmes composés de formule (II) où T est chlore, et Ar est Ar-1 (noyau phényle), peuvent être préparés par chloration des composés de formule (IV) au moyen du réactif $SO_2Cl_2/Na_2CO_3$ selon M. Bulliard et al. Tetrahedron Letters (1989), 30, 5767.

Les composés de formule (II) où T est chlore, Ar ayant la même définition que celle indiquée dans la définition générale de l'invention, peuvent également être préparés par chloration des composés de formule (IV) sus indiqués au moyen de N-chloro succinimide en présence de bisaryldisélénide suivant le procédé de K.B. Sharpless et T. Hori J. Org. Chem. (1979), 44, 4204.

Les composés de formule (II) où T est chlore ou brome, Ar ayant la même définition que celle indiquée dans la définition générale de l'invention, peuvent également être préparés par halogénation radicalaire thermique ou photochimique par la N-halogéno succinimide du composé de formule (IV), dans un solvant aprotique comme le tétrachlorure de carbone ou en l'absence de solvant avec ou non un initiateur de radicaux libres à une température de 20°C à 170°C (de préférence 80°C à 100°C) suivant S.F. Reed J. Org. Chem. (1965), 30, 3258. Ils peuvent encore être préparés par halogénation des composés de formule (II) où T est OH, Ar ayant la même définition que celle indiquée dans la définition générale de l'invention, avec un agent d'halogénation tel que $SOCl_2$, $POCl_3$, $PBr_3$, J. March ibid. p. 382-384 ou avec le réactif $LiCl/CH_3SO_2Cl$/collidine suivant E.W.

Collington et A.I. Meyers J. Org. Chem. (1971), 36, 3044.

Les composés de formule (II) où T est OH, Ar ayant la même définition que celle indiquée dans la définition générale de l'invention,peuvent être préparés par oxydation allylique du composé de formule (IV) par l'oxyde de sélénium catalytique ou non en présence d'un oxydant comme l'hydroperoxyde de tertiobutyle dans un solvant inerte comme les solvants halogénés (de préférence $CH_2Cl_2$) ou le tertiobutanol, en présence d'acide minéral ou organique selon M.A. Umbreit et K.B. Sharpless J. Amer. Chem. Soc. (1977), 99, 5526.

Les composés de formule (IV) peuvent être obtenus par déshydratation d'un composé 2-aryl 2-propanol de formule :

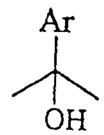

(V)

dans laquelle Ar, $R_1$ et m ou p ont la même signification que dans la définition générale de l'invention, par des agents de déshydratation comme l'acide paratoluènesulfonique, $P_2O_5$, $KHSO_4$, $POCl_3$/pyridine et d'autres selon J. March ibid. p. 901-903.

Les composés de formule (V) peuvent être préparés par mise en contact de l'acétophénone ou acétylpyridine ou dérivé d'acide de formule :

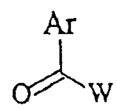

(VI)

dans laquelle Ar, $R_1$ et m ou p ont la même signification que dans la définition générale de l'invention et W est méthyle, alkoxy (ester benzoïque correspondant) ou chlore avec un ou deux équivalents d'halogénure de méthylmagnésium suivant J. March ibid. p. 816-822.

Les composés de formule (VI) sont obtenus de manière connue en soi.

Les composés de formule (III) peuvent être préparés par réduction des halogénures de sulfonyle correspondants (généralement chlorure) par le zinc, l'iodure de sodium ou de potassium, le sulfite de sodium selon J. March ibid. p. 445-446, et W.E. Truce et A.H. Murphy Chem. Rev. (1951), 48, 69. Les halogénures de sulfonyle peuvent être préparés selon J. March ibid. p. 1172.

Les composés de formule (III) peuvent être également préparés par action d'un organométallique (habituellement lithien) de formule :

$$M\,(CH_2)_f - B \quad (IIIA)$$

f, B ayant la même signification que celle indiquée dans la définition de l'invention, M étant notamment lithium, avec l'anhydride sulfureux $SO_2$, à une température comprise entre -78°C et 20°C (de préférence -78°C à -40°C) en l'absence ou en présence d'un solvant aprotique comme l'éther éthylique ou le tétrahydrofuranne selon H.W. Pinnick et M.A. Reynolds J. Org. Chem. (1979), 44, 160, et J. March ibid. p. 550.

Les composés de formule (IIIA) sont obtenus de manière connue en soi.

Les composés de formule (III) dans laquelle f = 0 et B est le groupe 1-alcényle peuvent être préparés par réaction de fragmentation de composés préalablement choisis selon K. Schank, R. Wilmes et G. Ferdinand Int. J. Sulfur Chem. (1973), 8, 397.

Méthode B

Les composés de formule (I) dans laquelle A est ($A_1$) et n = 0, 1 ou 2 peuvent être préparés par action du sel alcalin d'un thiolate d'hétéroaryle ou d'alcényle de formule :

$$M'\,S\,(CH_2)_f - B \quad (IIIB)$$

dans laquelle M' est un atome de métal alcalin ou alcalinoterreux notamment sodium ou potassium, f et B ayant la même définition que celle indiquée dans la définition de l'invention sur un composé de formule (II) où T est halogène précédemment décrit dans un solvant inerte protique ou aprotique tel que les cétones, les alcools, le tétrahydrofuranne, l'acétonitrile, à une température de 0°C à 80°C (généralement 25°C à 60°C) dans un rapport molaire II :IIIB compris généralement entre 1 et 10 (de préférence 1 et 2).

Le sulfure ainsi obtenu (n = 0) peut être oxydé en sulfoxyde (n = 1) par un équivalent d'oxydant à une température de -70°C à 5°C (généralement 0°C) ou être oxydé en sulfone (n = 2) par deux ou plus équivalents

d'oxydant à une température de 0°C à 60°C (généralement 10°C à 30°C) par de nombreux oxydants comme $KMnO_4$, $H_2O_2$, $CH_3CO_3H$, acides perbenzoïques, $KHSO_5$ et d'autres suivant de très nombreuses méthodes connues : J. March ibid. p. 1089-1090, et B.M. Trost et R. Braslau J. Org. Chem. (1988), 53, 532.

Méthode C

Les composés de formule (I) dans laquelle A est ($A_1$), n = 1 ou 2, f = 0 et B est le groupe 1-alcényle, les autres substituants ayant la même définition que celle indiquée dans la définition générale de l'invention peuvent être obtenus par mise en contact de l'anion d'un (2-aryl 2-propényl) sulfinométhylphosphonate de dialkyle (n=1) ou de l'anion d'un (2-aryl 2-propényl) sulfonométhylphosphonate de dialkyle (n=2) de formule :

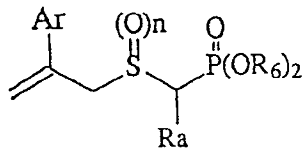

(VII)

dans laquelle $R_6$ est un groupe $C_1$ - $C_6$ alkyle, n = 1 ou 2, $R_a$ est hydrogène, halogène ou un groupe $C_1$ - $C_7$ alkyle, Ar ayant la même définition que dans la définition générale de l'invention, sur un aldéhyde $R_b$ (CH=O), $R_b$ étant un groupe $C_1$ - $C_8$ alkyle, ou sur une cétone $R_{b'}R_c$ (C=O), $R_{b'}$ étant un groupe $C_1$ - $C_7$ alkyle, $R_c$ étant un groupe $C_1$ - $C_7$ alkyle, les différents groupes alkyles $R_a$, $R_b$, $R_{b'}$ et $R_c$ pouvant être éventuellement ramifiés et éventuellement substitués par 1 à 6 atomes d'halogènes.

La réaction est effectuée dans un rapport molaire VII : $R_b$ (CH=O) ou $R_{b'}R_c$ (C=O) compris généralement entre 1 et 5 (de préférence 1 et 2), dans un solvant aprotique comme le tétrahydrofuranne en présence ou non de solvant aprotique dipolaire comme l'hexaméthylphosphotriamide, à une température de -78°C à 50°C (de préférence -78°C à 0°C) selon H.Fillion et al. J.Heterocyclic Chem.(1978), 15, 753. Les anions de formule VII où n=1 ou 2, sont préparés par action d'une base comme les hydrures alcalins, le butyllithium ou le diisopropylamidure de lithium. Les composés de formule VII dans laquelle n=1 ou 2, Ar ayant la même définition que dans la définition générale de l'invention et $R_a$ est un groupe $C_1$ - $C_7$ alkyle peuvent être obtenus par action de l'anion du composé de formule VII dans laquelle n=1 ou 2 et $R_a$ est hydrogène, sur un halogènure $R_a$Hal où $R_a$ est le groupe $C_1$ - $C_7$ alkyle et Hal un halogène (généralement brome) selon P.O. Ellingsen et K. Undheim Acta Chem. Scand. Ser. B (1979), B 33, 528.

Les composés de formule VII dans laquelle n =1 ou 2, Ar ayant la même définition que dans la définition générale de l'invention et $R_a$ est un atome d'halogène (notamment chlore ou brome) peuvent être obtenus par action de l'anion du composé de formule VII dans laquelle n = 1 ou 2 et $R_a$ est hydrogène, sur un donneur d'halogène comme la N-halogéno succinimide, le chlorure de sulfuryle, le tétrachlorure de carbone, l'hexachloroéthane et d'autres suivant E.R. de Waard et al. Tetrahedron (1980), 36, 1847 ou E.Bertele et P.Enggist Helv. Chim. Acta (1971), 54, 456.

Les composés de formule VII dans laquelle n = 1 ou 2, Ar ayant la même définition que dans la définition générale de l'invention et $R_a$ est hydrogène ou un groupe $C_1$ - $C_7$ alkyle, peuvent être obtenus par oxydation par un équivalent d'oxydant (n =1, sulfoxyde) ou deux équivalents d'oxydant (n = 2, sulfone) des composés de formule VII dans laquelle n = 0, Ar ayant la même définition que dans la définition générale de l'invention et $R_a$ est hydrogène ou un groupe $C_1$ - $C_7$ alkyle, suivant les méthodes décrites dans la méthode B.

Les composés de formule VII dans laquelle n = 0, Ar ayant la même définition que dans la définition générale de l'invention et $R_a$ est hydrogène ou un groupe $C_1$ - $C_7$ alkyle, peuvent être obtenus par action du sel alcalin d'un thiolométhylphosphonate de dialkyle de formule :

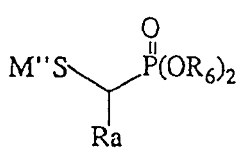

(VIII)

dans laquelle M" est un atome de métal alcalin (notamment sodium), $R_6$ est groupe $C_1$ - $C_6$ alkyle et $R_a$ est hydrogène ou un groupe $C_1$ - $C_7$ alkyle, sur un composé de formule (II) où T est halogène précédemment décrit, suivant les conditions décrites dans la méthode B.

Les composés de formule (VIII) sont obtenus suivant des méthodes bien connues (C.K. Farrington et al. J. Med. Chem. (1985), 28, 1668.)

Méthode D

Les composés de formule (I) dans laquelle A est ($A_1$), et n = 2, les autres substituants ayant la même définition que celle indiquée dans la définition générale de l'invention peuvent être obtenus dans une première étape, par mise en contact de l'anion ou dianion d'une méthylhétéroarylsulfone ou méthylalcénylsulfone de formule :

$$CH_3\,SO_2\,(CH_2)_f - B \quad (IX)$$

f, B ayant la même définition que celle indiquée dans la définition générale de l'invention, sur une acétophénone ou une acétylpyridine de formule (VI) dans laquelle W est méthyle et Ar, $R_1$ et m ou p ont la même définition que dans la définition générale de l'invention pour fournir les composés de formule :

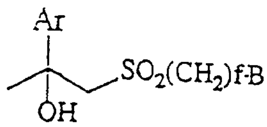

(X)

dans laqelle Ar, $R_1$, m ou p, f et B ont la même signification que dans la définition générale de l'invention.

La réaction est effectuée dans un rapport molaire VI : IX compris généralement entre 1 et 5 (de préférence 1 et 2), dans un solvant aprotique comme le tetrahydrofuranne à une température de -70°C à 50°C (de préférence -70°C à 20°C) selon N. Hanack et K. Laping, Tetrahedron Letters (1977), 4493 ou D.F. Tavares et P.F. Vogt Can. J. Chem. (1967), 45, 1519.

Les anions des composés de formule (IX) sont préparés par action d'un ou plus équivalents d'organométallique comme le butyllithium, le diisopropylamidure de lithium ou les halogénures d'alkylmagnésium sur les composés correspondants.

Les composés de formule (I) dans laquelle A est ($A_1$) et n = 2 sont obtenus dans une deuxième étape, par déshydratation des composés de formule (X) suivant la méthode décrite dans la méthode A pour la déshydratation des composés de formule (V).

La déshydratation fournit un mélange du composé de formule (I) dans laquelle A est ($A_1$) et n = 2, et éventuellement du composé de formule (XI)

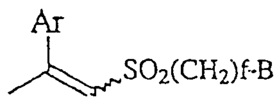

(XI)

dans laquelle Ar, $R_1$, m, p, f et B ont la même signification que dans la définition générale de l'invention. Ces composés de formule (XI) peuvent être isomérisés en composés de formule (I) dans laquelle A est ($A_1$) et n = 2, en milieu basique par action de 1 à 5 équivalents de base (de préférence 2) comme le tertiobutylate de potassium, le 1,8-diazabicyclo [5,4,0] undéc-7-ène dans des solvants comme l'acétonitrile, le diméthylsulfoxyde, le terbutanol à une température de 25°C à 100°C (de préférence 25°C à 60°C) selon D.E O'Connors et W.I.Lyness J.Amer.Chem. Soc. (1964), 86, 3044 ou T.Kobayashi et al. Chem. Letters (1987), 1209.

Les méthylhétéroarylsulfones ou méthylalcénylsulfones de formule (IX) sont préparés par action d'un sulfinate d'hétéroaryle ou d'alcényle de formule (III) sur l'iodure de méthyle ou le diméthyl sulfate, ou par oxydation des méthylhétéroarylesulfures ou méthylalcénylsulfures correspondants préparés par action d'un thiolate d'hétéroaryle ou d'alcényle de formule (IIIB) sur l'iodure de méthyle ou le diméthylsulfate, suivant les méthodes décrites dans les méthodes A et B.

Les méthylhétéroarylsulfones de formule (IX) peuvent également être préparés par méthylsulfonylation directe du noyau aromatique correspondant, par l'anhydride méthanesulfonique en présence d'acide trifluorométhanesulfonique catalytique selon M.Ono et al. Chem. Letters (1988), 395, ou par le fluorure de méthylsulfonate en présence de chlorure d'aluminium selon J.A.Hyatt et A.W.White Synthesis (1984), 214.

Méthode E

Les composés de formule (I) dans laquelle A est ($A_1$), et n = 2, les autres substituants ayant la même définition que celle indiquée dans la définition générale de l'invention peuvent être obtenus dans une première étape, par mise en contact de l'anion ou dianon d'une méthylhétéroarylsulfone ou méthylalcénylsulfone de formule (IX) dans laquelle f et B ont la même définition que celle indiquée dans la définition générale, sur une

2-silylacétophénone ou α-silylacétylpyridine de formule :

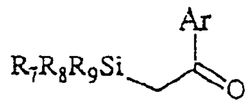

(XII)

dans laquelle Ar, $R_1$, m ou p ont la même signification que dans la définition générale de l'invention et $R_7$, $R_8$, et $R_9$ sont des groupements alkyles ou phényles, pour fournir les composés de formule :

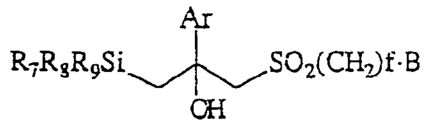

(XIII)

dans laquelle Ar, $R_1$, m ou p, $R_7$, $R_8$ et $R_9$ ont les définitions sus indiquées.

La réaction est effectuée dans les conditions indentiques à celles décrites pour la préparation des composés de formule (X) dans la méthode D.

Les composés de formule (I) dans laquelle A est ($A_1$) et n = 2 sont obtenus dans une deuxième étape, par désilylation des composés de formule (XIII) par de très nombreux réactifs comme $BF_3$ :$Et_2O$, tBuOK, $H_2SO_4$, AcOH/AcONa, dans un rapport molaire XIII : réactif compris généralement entre 1 et 10 (de préférence 1 et 5) à une température de - 70°C à 100°C (de préférence 0°C à 50°C) dans de nombreux solvants comme les solvants chlorés, l'acide acétique, le tétrahydrofuranne selon D.J. Ager Synthesis (1984), 384.

Les composés de formule (XII) sont préparés suivant des méthodes en soi connues.

Méthode F

Les composés de formule (I) dans laquelle A est ($A_3$), Y est Cl ou Br, Z est H, les autres substituants ayant la même définition que celle indiquée dans la définition générale de l'invention peuvent être obtenus par halogénation en milieu aqueux des composés précédemment obtenus de formule (I) où A est ($A_1$). Les agents d'halogénation sont la N-halogéno succinimide, le N-halogéno acétamide dans un rapport molaire I : agent d'halogénation de 1 à 10 (de préférence 2 à 4) dans un mélange de proportion 5/95 à 90/10 (de préférence 5/95 à 20/80) d'eau et d'un solvant soluble dans l'eau comme le diméthylsulfoxyde, le dioxanne, l'acétone et autres à une température de 0°C à 60°C (de préférence 20°C à 40°C) en présence ou non d'un acide qui peut être par exemple l'acide acétique ou l'acide trifluoroacétique en présence ou non d'une quantité catalytique ou non d'un sel de lithium comme les halogénures, le perchlorate, le tétrafluoroborate, selon H.O. House J. Amer. Chem. Soc. (1955), 77, 3070 ou J. March ibid. p. 726-727.

Méthode G

Les composés de formule (I) dans laquelle A est ($A_3$), Y est Cl, Br, I, Z est H, peuvent être préparés par substitution nucléophile des composés de formule (I) dans laquelle A est ($A_3$), Y est $OSO_2R_2$, Cl, Br, Z est H, par le sel alcalin (sodium, potassium, lithium) d'halogénure (Y = Cl, Br, I) en rapport molaire I : sel de 1 à 10 (de préférence 1 à 3) dans des solvants comme l'acétone, la méthyléthylcétone, le glyme, le diglyme, le diméthylformamide, à une température de 25°C à 180°C (de préférence 40°C à 160°C) selon J. March ibid. p. 381-382).

Méthode H

Les composés de formule (I) dans laquelle A est ($A_3$), Y est Cl ou Br, Z est H et n = 2, les autres substituants ayant la même définition que celle indiquée dans la définition générale de l'invention, peuvent être préparés par mise en contact de l'anion d'une méthylhétéroarylsulfone ou méthylalcénylsulfone de formule (IX), f et B ayant la même définition que celle indiquée dans la définition générale de l'invention, sur une 2-halogénoacétophénone ou halogénoacétylpyridine de formule :

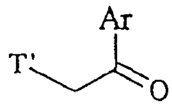 (XIV)

dans laquelle T′ est un atome de chlore ou de brome, Ar, $R_1$, m ou p ayant la même signification que celle indiquée dans la définition générale de l'invention, dans un rapport molaire XIV :IX compris généralement entre 1 et 5 (de préférence 1 et 2), dans un solvant aprotique comme le tétrahydrofuranne, le diméthoxyéthane à une température de -100°C à 0°C (de préférence - 70°C à - 20°C) selon les références décrites dans la méthode D.

Les anions des composés de formule (IX) et les composés de formule (IX) sont préparés suivant les méthodes citées dans la méthode D.

Les composés de formule (XIV) sont préparés suivant des méthodes en soi connues.

Méthode I

Les composés de formule (I) dans laquelle A est ($A_3$), Y est Br ou Cl, Z est H, les autres substituants ayant la même définition que celle indiquée dans la définition générale de l'invention, peuvent être préparés par ouverture des composés de formule (I) dans laquelle A est ($A_2$), X est O, les autres substituants ayant la même définition que celle indiquée dans la définition générale de l'invention, par action d'un réactif donneur d'halogénure $Y^-$, Y étant l'atome de chlore ou de brome, comme les acides halohydriques HY, l'halogénure de magnésium $MgY_2$, les complexes $NiLi_2Y_4$ selon R.D. Dawe, T.F. Molinski et J.V. Turner Tetrahedron Letters (1984), 25, 2061 ou J. March ibid., p. 385-386.

Méthode J

Les composés de formule (I) dans laquelle A est ($A_3$), Y est Br ou Cl, Z est ester, les autres substituants ayant la même définition que celle indiquée dans la définition générale de l'invention, peuvent être préparés par halogénation par la N-halogéno succinimide, le N-halogéno acétamide, des composés précédemment obtenus de formule (I) où A est ($A_1$) en présence d'un acide $R_5CO_2H$, $R_5$ ayant la même définition que celle indiquée dans la définition générale de l'invention, dans un rapport molaire I : agent d'halogénation de 1 à 10 (de préférence 1 à 3), dans un mélange de proportion 100/0 à 10/90 (de préférence 100/0 à 50/50) d'acide et d'un solvant comme l'acétone, le dioxanne et autres à une température de 0°C à 60°C (de préférence 0°C à 20°C) en présence ou non du sel de l'acide $R_5CO_2M$, M étant l'atome de sodium, potassium ou lithium dans un rapport molaire I : sel d'acide de 1 à 10 (de préférence 5) selon V.L. Heasley et R.A. Skidgel J. Org. Chem. (1974), 89, 3953.

Les composés de formule (I) dans laquelle A est ($A_3$), Y est I, Z est ester, peuvent être préparés par substitution nucléophile des composés de formule (I) où A est ($A_3$), Y est Br, Z est ester suivant la méthode décrite dans la méthode G.

Méthode K

Les composés de formule (I) dans laquelle A est ($A_3$), Y est $OSO_2R_2$, Z est H, les autres substituants ayant la même définition que celle indiquée dans la définition générale de l'invention peuvent être obtenus par action de l'halogénure $R_2SO_2$-Hal (Hal étant de préférence l'atome de chlore) sur le composé diol de formule :

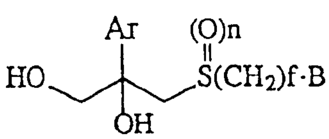 (XV)

en présence d'une base azotée comme la pyridine, la triéthylamine dans un rapport molaire XV : halogénure: base compris entre 1 :1 :1 et 1 :5 :100 (de préférence 1 :1 :1 et 1 :2 :5) dans un solvant comme l'éther, le diméthoxyéthane, le tétrahydrofuranne et autres selon J. March ibid. p. 357-358.

Le composé de formule (XV) peut être préparé par perhydroxylation des composés de formule (I) où A est ($A_1$) (n = 1, 2) suivant des méthodes connues (J. March ibid. p. 732-734) comme par exemple l'action catalytique

du tétraoxyde d'osmium en présence d'un oxydant comme l'hydroperoxyde de tertiobutyle dans un solvant inerte comme l'acétone ou autre en présence ou non d'un sel d'ammonium selon Akaski, R.E. Palermo et K.B. Sharpless J. Org. Chem. (1978), 43, 2063.

Méthode L

Les composés de formule (I) dans laquelle A est ($A_2$), X est O, les autres substituants ayant la même signification que celle indiquée dans la définition générale de l'invention peuvent être obtenus par cyclisation d'un composé de formule (I) où A est ($A_3$) et Y est Cl, Br, I, Z est H, en présence de sel d'argent $Ag_2O$ dans un rapport molaire I : $Ag_2O$ compris entre 1 et 5 (de préférence 2 et 3) dans un solvant aprotique comme l'éther, le tétrahydrofuranne, le diméthoxyéthane à une température de 25°C à 150°C (de préférence 60°C à 85°C) suivant J.D. Mc Clure J. Org. Chem. (1967), 32, 3888.

Dans le cas où n = 0 ou 1, ces mêmes composés de formule (I) dans laquelle A est ($A_2$) peuvent également être préparés par cyclisation en milieu basique (NaOH, KOH, $K_2CO_3$ et autres) des composés de formule (I) où A est ($A_3$), Y est Cl, Br ; I, $OSO_2R_2$, Z est H, selon J. March ibid. p. 343.

Les composés de formule (I) où A est ($A_2$) et n = 2 ou 1 peuvent être préparés par oxydation des composés de formule (I) où A est ($A_2$) et n = 1 ou 0 suivant les méthodes décrites dans la méthode B.

Méthode M

Les composés de formule (I) dans laquelle A est ($A_2$), n = 2, X = O, les autres substituants ayant la même signification que celle indiquées dans la définition générale de l'invention peuvent être obtenus par oxydation d'un composé de formule (I) où A est ($A_1$), n = 2, par des agents d'oxydation comme les peracides organiques dans un rapport molaire I : oxydant compris entre 1 et 10 (de préférence 1 à 3) en milieu tamponné par le sel alcalin ou alcalino-terreux d'un acide organique selon J. March ibid. p. 735-736.

Méthode N

Les composés de formule (I) où A est ($A_2$) et X = O, les autres substituants ayant la même signification que celle indiquées dans la définition générale de l'invention, peuvent être préparés par déshydratation des composés de formule (XV), par des agents de déshydratation comme le diméthylacétaldiméthylformamide, le réactif diéthylazodicarboxylate/triphénylphosphine selon C. Cortez et R.G. Harvey, Org. Synth. (1978), 58, 12, ou J.T. Carlock et M.P. Mack Tetrahedron Letters (1978), 5153.

Méthode O

Les composés de formule (I) où A est ($A_2$) et X = S, les autres substituants ayant la même définition que celle indiquée dans la définition générale de l'invention, sont préparés par réaction des composés de formule (I) où A est ($A_2$) et X est O avec 1 à 10 équivalents (de préférence 1 à 3) d'un sel alcalin (généralement potassium) du thiocyanate dans des solvants comme l'éthanol, l'acétonitrile et autres à des températures de 25°C à 120°C (de préférence 25°C à 60°C) selon Org. Synth., IV, 232.

Ces mêmes composés peuvent être également préparés d'après de nombreuses autres méthodes connues J. March ibid. p. 362.

Méthode P

Les composés de formule (I) dans laquelle n = 0, 1, 2, et A est ($A_4$), les autres substituants ayant la même définition que celle indiquée dans la définition générale de l'invention peuvent être obtenus par mise en contact d'un composé de formule (I) pour laquelle A est ($A_1$), les autres substituants ayant la même signification que dans la définition générale de l'invention, avec des halogénures UV comme le chlore (U = Cl, V = Cl), le brome (U = Br, V = Br),l'iodure de chlore (U = Cl, V = I), l'iodure de brome (U = Br, V = I) ou le bromure de chlore (U = Cl, V = Br), dans un solvant inerte aprotique comme le chloroforme, le tétrachlorure de carbone, le tétrahydrofuranne, le diméthoxyéthane, l'acétonitrile en présence ou non d'un acide comme l'acide acétique ou l'acide chlorhydrique à une température de - 78°C à 60°C (de préférence 0°C à 20°C) et dans un rapport molaire I : UV compris entre 1 et 5 (de préférence 1 et 2). Cette réaction est notamment connue par J. March ibid. p. 724-726, S. Akiyoshi et K. Okuno J. Amer. Chem. Soc. (1952), 74, 4283 et F.G. Weber Tetrahedron (1969), 25, 4283.

Ces mêmes composés de formule (I) dans laquelle A est ($A_4$), n = 0, 1 ou 2, les autres substituants ayant

la même définition que celle indiquée dans la définition générale de l'invention peuvent également être préparés par action sur un composé de formule (I) dans laquelle A est ($A_1$), les autres substituants ayant la même signification que dans la définition générale de l'invention, d'un agent d'halogénation $R_{10}V$, V étant l'atome de chlore ou de brome et $R_{10}$ un radical acétamido comme le N-halogéno acétamide, la N-halogéno succinimide en présence d'un donneur d'anion halogénure $U^-$, U étant l'atome de chlore ou de brome, comme les acides halohydriques HU, les halogénures d'ammonium $(R_{11})_4NU$, $R_{11}$ étant un radical alkyle ou les sels MU, M étant un atome de métal alcalin, alcalino-terreux ou l'atome d'argent dans un solvant inerte comme le chlorure de méthylène, le chloroforme, l'acétonitrile, le diméthoxyéthane ou l'acide acétique à une température de - 78°C à 60°C (de préférence 0°C à 20°C) et dans un rapport molaire I : $R_{10}V$ : $U^-$, compris entre 1 : 1 : 1 et 1 : 5 : 100 (de préférence 1 : 2 : 5). Cette réaction est notamment connue par J. March ibid. p. 725, R.E. Buckles et J.W. Long J. Amer. Chem. Soc. (1959), 81, 2191, A. Marquet et J. Jacques Tetrahedron Letters (1959), 9, 24 et C.H. Robinson et al. J. Amer. Chem. Soc. (1959), 81, 2191.

Les composés de formule (I) dans laquelle n = 0 ou 1, A est ($A_4$), les autres substituants ayant la même définition que celle indiquée dans la définition générale de l'invention, peuvent être oxydés en sulfoxyde (n = 1) par un équivalent d'oxydant à un température de - 70°C à 5°C (généralement 0°C) ou être oxydés en sulfone (n = 2) par deux ou plus équivalents d'oxydant à une température de 0°C à 60°C (généralement 10°C à 30°C) par de nombreux oxydants comme $KMnO_4$, $H_2O_2$, $CH_3CO_3H$, acides perbenzoïques, $KHSO_5$ et d'autres suivant de très nombreuses méthodes connues J. March ibid. p. 1089-1090 et B.M. Trost et R. Braslau J. Org. Chem. (1988), 53, 532.

L'invention a également pour objet les produits (II) à (XV) nouveaux utiles pour la mise en oeuvre du procédé qui vient d'être décrit.

Les exemples suivants illustrent l'invention :

Exemple 1

On dissout 130 $cm^3$ (1 mole) de 2-phényl 1-propène dans 1 500 $cm^3$ de chlorure de méthylène. On ajoute 300 $cm^3$ d'eau et 101,5 g (0,5 mole) d'hypochlorite de calcium à 70 % en chlore actif. Sous très forte agitation, on additionne pendant 2 heures de la carboglace. On décante les deux phases et sèche la phase organique sur $MgSO_4$. Après évaporation, on obtient 145 g d'une huile jaune. L'analyse RMN (60 MHz) révèle la présence de 45 % de 1-chloro 2-phényl 2-propène, 45 % de 1-chloro 2-phényl 1-propène et 10 % de 1-chloro 2-phényl 2-propanol.

27,1 g de ce mélange (0,08 mole en 1-chloro 2-phényl 2-propène) sont additionnés goutte-à-goutte en solution acétonique (80 $cm^3$) et en mélange avec 9,1 g (0,08 mole) de 2-méthanethiolfuranne à 12,4 g (0,09 mole) de carbonate de potassium en suspension dans 280 $cm^3$ d'acétone à 55°C. Après 3 heures à 55°C le milieu réactionnel est versé dans 250 $cm^3$ d'eau et réextrait par 3 X 200 $cm^3$ d'éther. La phase éthérée est lavée jusqu'à neutralité et séchée sur $MgSO_4$.

L'évaporation laisse 23,3 g d'une huile qui est distillée au four à boules (Pression P = 0,3 mm Hg ; Température T = 150-160°C) pour donner 10,4 g (56 %) de 1-[(2-furyl) méthylthio] 2-phényl 2-propène. $n_D^{24}$ = 1,623.

Exemple 2

8,2 g (0,035 mole) de 1-[(2-furyl) méthylthio] 2-phényl 2-propène sont dissous dans 200 $cm^3$ de méthanol et refroidis à 0°C. On ajoute 23,4 g (0,076 mole en persulfate de potassium) d'Oxone® en solution dans 200 $cm^3$ d'eau. On laisse revenir à température ambiante et agite pendant un jour. Le milieu réactionnel est versé dans 300 g d'eau et de glace et est fortement agité avec 50 $cm^3$ de pentane jusqu'à cristallisation. Les cristaux sont filtrés et séchés sur $P_2O_5$ pour donner 6,6 g (72 %) de 1-[(2-furyl) méthylsulfonyl] 2-phényl 2-propène. F = 95°C.

Exemple 3

On dissout 34 $cm^3$ (0,35 mole) de 2-bromothiophène dans 500 $cm^3$ d'éther anhydre et refroidit sous argon à - 70°C. On ajoute goutte-à-goutte 220 $cm^3$ (0,35 mole) d'une solution 1,6 molaire de butyllithium dans l'heptane en 30 minutes, sans dépasser - 60°C. La suspension blanche est encore agitée pendant 1 H 30 à - 70°C.

Sous forte agitation, 65 g (1 mole) d'anhydride sulfureux liquide sont ajoutés progressivement. On laisse revenir à température ambiante, filtre le solide, lave par 3 X 200 $cm^3$ d'éther et essore à fond. Après séchage, on obtient 54 g (100 %) de cristaux blanc cassé de 2-thiénylsulfinate de lithium.

On dissout 68 g (0,5 mole) de 2-(3-fluorophényl)1-propène dans 1000 $cm^3$ de chlorure de méthylène. On ajoute 212g (2 moles) de carbonate de sodium et refroidit à 0°C. Sous très forte agitation, on additionne en 2

heures, 41 $cm^3$ (0,5 mole) de chlorure de sulfuryle distillé en solution dans 500 $cm^3$ de chlorure de méthylène. L'agitation est maintenue pendant encore 2 heures. Après filtration et évaporation, on obtient 82g d'une huile jaune. L'analyse RMN (60 MHz) révèle la présence de 49% de 1-chloro 2-(3-fluorophényl)2-propène, 41% de 1-chloro 2-(3-fluorophényl)1-propène et 10% de 1,2-dichloro 2-(3-fluorophényl)propane.

1,5 g (0,01 mole) de 2-thiénylsulfinate de lithium sont ajoutés à 3,5 g de ce mélange comprenant 0,01 mole de 1-chloro 2-(3-fluorophényl 2-propène, en solution dans 50 $cm^3$ de diméthylformamide. Le milieu est chauffé 2 heures à 70°C, puis versé dans 100 g d'eau et de glace. La phase aqueuse est réextraite par 3 X 30 $cm^3$ d'éther et les extraits éthérés séchés sur $MgSO_4$. Une chromatographie sur silice du brut (éluant chloroforme/heptane : 40/60) donne 2,0 g (71%) de 2-(3-fluorophényl) 1-(2-thiénylsulfonyl) 2-propène. F = 45°C.

Exemple 4

On dissout 15,3 g (0,1 mole) de 2-(6-chloro 2-pyridyl) 1-propène dans 200 $cm^3$ de 1,2-dichloroéthane. On ajoute 0,5 g (catalytique) de bis(4-chlorophényl) disélénide et 14,7 g (0,11 mole) de N-chlorosuccinimide et chauffe 24 heures à 60°C. On concentre le milieu réactionnel au tiers, filtre le succinimide, et lave la phase organique par 2 x 200 $cm^3$ d'eau, 1 x 200 $cm^3$ d'une solution à 15% de bicarbonate de sodium, 2 x 200 $cm^3$ d'eau et sèche sur $MgSO_4$. Brut : 13,9 g.

L'analyse RMN (60 MHz) révèle la présence de 55 % de 1-chloro 2-(6-chloro 2-pyridyl) 2-propène et 45 % de 1-chloro 2-(6-chloro 2-pyridyl) 1-propène.

A 5,5 g (0,015 mole en 1-chloro 2-(6-chloro 2-pyridyl) 2-propène) de ce mélange en solution dans 30 $cm^3$ d'acétonitrile est ajouté 2,3 g (0,015 mole) de 2-thiénylsulfinate de lithium et 1,1 g (0,0075 mole) d'iodure de sodium en solution dans 10 $cm^3$ d'eau. Le milieu est chauffé à 75°C pendant 2 heures.

Après refroidissement, on ajoute 20 $cm^3$ de saumure, sépare les deux phases et réextrait la phase aqueuse par 3 X 30 $cm^3$ de dichlorométhane. Les extraits organiques sont lavées par 3 X 30 $cm^3$ d'eau et séchés sur $MgSO_4$.

L'évaporation laisse 6,7 g d'une huile qui cristallise. Après recristallisation dans 75 $cm^3$ d'éthanol, on obtient 2,8 g (62 %) de cristaux de 2-(6-chloro 2-pyridyl) 1-(2-thiénylsulfonyl) 2-propène. F = 131°C.

Exemple 5

D'une manière identique à l'exemple 4, on traite 10,5 g d'un mélange comprenant 0,03 mole de 1-chloro 2-(3-fluorophényl) 2-propène préparé suivant l'exemple 3, par 6,2 g (0,03 mole) de 3-chloro 2-thiénylsulfinate de sodium en présence de 2,2 g (0,015 mole) d'iodure de sodium dans l'acétonitrile aqueux à 75 % à 75°C pendant 2 heures. Le même traitement donne après recristallisation dans l'éthanol, 2,4 g (25%) de 1-[(3-chloro 2-thiényl) sulfonyl] 2-(3-fluorophényl) 2-propène. F = 47°C.

Exemple 6

On ajoute goutte-à-goutte à température ambiante, 2,9 g (0,018 mole) de 2-méthylsulfonylthiophène en solution dans 30 $cm^3$ de tétrahydrofuranne anhydre, à 0,022 mole d'une solution 0,5 molaire de bromure d'éthylmagnésium dans l'éther. Le milieu est agité 1 h à température ambiante jusqu'à fin du dégagement gazeux.

En maintenant la température à 5°C à l'aide d'un bain de glace, on ajoute goutte-à-goutte 2,8 g (0,018 mole) de 3',5'-difluoroacétophénone en solution dans 15 $cm^3$ de tétrahydrofuranne anhydre. Après 1 h d'agitation, le milieu est hydrolysé par 1 équivalent d'acide acétique, dilué par 100 $cm^3$ d'eau et réextrait par 3 x 50 $cm^3$ d'éther. Les extraits éthérés sont lavés à l'eau et séchés sur $MgSO_4$. L'évaporation laisse 5,6 g d'une huile qui cristallise. Après recristallisation dans 10 $cm^3$ d'éthanol, on obtient 1,8 g (31 %) de cristaux blancs de 2-(3,5-difluorophényl) 1-(2-thiénylsulfonyl) 2-propanol. F = 93 °C.

Exemple 7

On dissout 1,8 g de 2-(3,5-difluorophényl) 1-(2-thiénylsulfonyl) 2-propanol dans 50 $cm^3$ de toluène et ajoute 180 mg (10 % en poids) d'acide paratoluènesulfonique. Le milieu est porté à reflux pendant 1 h. Après refroidissement la phase toluènique est lavée jusqu'à neutralité et séchée sur $MgSO_4$ L'évaporation laisse 1,8 g d'une huile qui est cristallisée dans 5 $cm^3$ d'éthanol pour donner 1,0 g (60 %) de 2-(3,5-difluorophényl) 1-(2-thiénylsulfonyl) 2-propène. F = 72 °C

Exemple 8

12,6 g (0,06 mole) de 3'-fluoro 2-triméthylsilylacétophénone en solution dans 40 $cm^3$ de tétrahydrofuranne anhydre sont ajoutés goutte-à-goutte à -20°C à 0,06 mole de l'anion magnésié du 3-bromo 2-méthylsulfonylthiophène en solution 0,5 molaire dans l'éther, préparé comme dans l'exemple 6. Après 1 heure d'agitation à -20°C, le milieu réactionnel est traité comme dans l'exemple 6 pour donner 16,1 g de cristaux blancs. Une recristallisation dans 25 $cm^3$ d'acétone donne 7,2 g (30 %) de cristaux de 1-[(3-bromo 2-thiényl) sulfonyl] 2-(3-fluorophényl) 3-triméthylsilyl 2-propanol. F = 122°C.

Exemple 9

7,2 g (0,018 mole) de 1-[(3-bromo 2-thiényl) sulfonyl] 2-(3-fluorophényl) 3-triméthylsilyl 2-propanol sont dissous dans 100 $cm^3$ de chlorure de méthylène et refroidis à 0°C. On ajoute 11 ml (0,09 mole) de $BF_3$-éthérate et agite 1 heure à température ambiante. La phase organique est lavée à l'eau jusqu'à neutralité, séchée sur $MgSO_4$ et évaporée pour donner 5,4 g d'une huile incolore. Par trituration dans 20 $cm^3$ d'éthanol, on obtient 3,8 g (60 %) de cristaux de 1-[(3-bromo 2-thiényl)sulfonyl] 2-(3-fluorophényl)2-propène. F = 65°C.

Les exemples suivants sont préparées suivant les méthodes des exemples 1 à 9

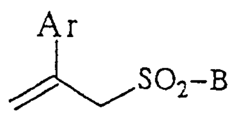

| Exemple | Ar- | R1 | B | F (Solvant) nd (Température) |
|---|---|---|---|---|
| 10 | Ar-1 | H | 2-Me 3-furyl | 95°C ( C ) |
| 11 | Ar-1 | H | 2-thiényl | 94°C ( EtOH ) |
| 12 | Ar-1 | H | 2-thiazolyl | 80°C |
| 13 | Ar-1 | 3-Cl | 2-thiényl | 96°C ( C ) |
| 14 | Ar-1 | 3-CF3 | 2-thiényl | 55°C ( EtOH ) |
| 15 | Ar-1 | 3,5-diCl | 2-thiényl | 107°C |
| 16 | Ar-1 | 3-Cl | 3-Me 2-thiényl | 1,6075 ( 24°C ) |
| 17 | Ar-1 | 3-F | 3-Me 2-thiényl | 52°C ( C ) |
| 18 | Ar-1 | 3-Br | 3-Me 2-thiényl | 1,6140 ( 24°C ) |
| 19 | Ar-1 | H | 3-thiényl | 97°C ( EtOH ) |
| 20 | Ar-1 | 3-Cl | 3-thiényl | 76°C ( EtOH ) |
| 21 | Ar-1 | 3-F | 3-thiényl | 1,5920 (24°C ) |
| 22 | Ar-1 | 3-Cl 4-F | 3-thiényl | 84°C ( C ) |
| 23 | Ar-1 | 3-Me 4-F | 3-thiényl | 83°C ( C ) |
| 24 | Ar-1 | H | 2-Me 3-thiényl | 90°C ( EtOH ) |
| 25 | Ar-1 | H | 2-Cl 3-thiényl | 107°C ( EtOH ) |
| 26 | Ar-1 | 3-F | 2-Cl 3-thiényl | 43°C (C) |
| 27 | Ar-1 | H | 2,5-diCl 3-thiényl | 64°C ( EtOH ) |
| 28 | Ar-1 | 3-F | 2,5-diCl 3-thiényl | 78°C ( EtOH ) |
| 29 | Ar-1 | 3-CF3 | 3-Me 2-thiényl | 42°C ( C ) |
| 30 | Ar-1 | H | 3-Cl 2-thiényl | 100°C ( EtOH ) |
| 31 | Ar-1 | 3-NO2 | 2-thiényl | 106°C ( EtOH ) |
| 32 | Ar-2 | 6-CL | 3-Me 2-thiényl | 144°C ( EtOH ) |

| Exemple | Ar- | R1 | B | F (Solvant) nd (Température) |
|---|---|---|---|---|
| 33 | Ar-2 | 6-Cl | 3-Cl 2-thiényl | 164°C ( Acétone ) |
| 34 | Ar-2 | 6-F | 3-Cl 2-thiényl | 127°C ( Acétone ) |
| 35 | Ar-3 | 5-Br | 3-Cl 2-thiényl | 128°C ( EtOH ) |
| 36 | Ar-1 | 3-Me 4-F | 3-Cl 2-thiényl | 82°C ( EtOH ) |
| 37 | Ar-1 | 3-Me | 3-Cl 2-thiényl | 107°C ( Cyclohexane ) |
| 38 | Ar-1 | H | 3-Br 2-thiényl | 91°C ( EtOH ) |
| 39 | Ar-1 | 3-Me | 3-Br 2-thiényl | 95°C ( EtOH ) |
| 40 | Ar-1 | 3-Cl | 3-Br 2-thiényl | 83°C ( Et2O ) |
| 41 | Ar-1 | 3-Me | 3-Me 2-thiényl | 1,5828 (21°C) |
| 42 | Ar-1 | H | 4-Me 5-thiazolyl | 96°C ( EtOH ) |
| 43 | Ar-1 | H | 2,4-diMe 5-thiazolyl | 77°C ( C ) |
| 44 | Ar-1 | 3-Cl | 2,4-diMe 5-thiazolyl | 89°C ( C ) |
| 45 | Ar-1 | 3-F | 2,4-diMe 5-thiazolyl | 87°C ( C ) |
| 46 | Ar-1 | 3,5-diMe | 2,4-diMe 5-thiazolyl | 99°C ( EtOH ) |
| 47 | Ar-1 | 3-Me | 2,4-diMe 5-thiazolyl | 73°C ( EtOH ) |
| 48 | Ar-1 | 3-CF3 | 2,4-diMe 5-thiazolyl | 37°C ( C ) |
| 49 | Ar-1 | 3-Br | 2,4-diMe 5-thiazolyl | 68°C ( C ) |
| 50 | Ar-1 | 3-Et | 2,4-diMe 5-thiazolyl | 1,5821 (20°C) |
| 51 | Ar-1 | H | 2,5-diMe 3-thiényl | 1,5900 (22°C) |
| 52 | Ar-1 | 3-Me | 2,5-diMe 3-thiényl | 1,5907 (20°C) |

C = Chromatographie

Avec Ar-1 = phényl
Ar-2 = 2-pyridyl
Ar-3 = 3-pyridyl

## Exemple 53

On dissout 2 g (0,007 mole) de 2-(3-fluorophényl) 1-(2-thiénylsulfonyl) 2-propène dans 30 $cm^3$ d'acétone. On ajoute successivement 30 $cm^3$ d'acide trifluoroacétique, 5 $cm^3$ d'eau et 75 mg (0,1 équivalent) de perchlorate de lithium. Le mélange est refroidi à 0°C et on ajoute en une seule fois, 1,4 g (0,008 mole) de N-bromoacétamide (pureté 82 %). Le milieu est agité une nuit à température ambiante, puis versé dans 100 g d'un mélange eau et glace. On réextrait par 3 X 30 $cm^3$ de dichlorométhane, lave la phase organique par 2 X 50 $cm^3$ d'eau et sèche sur $MgSO_4$.

L'évaporation laisse 3,3 g d'une huile jaune qui cristallise en solution éthérée à 5°C. Une recristallisation dans un mélange 50/50 d'éther et de chloroforme, fournit 0,7 g (27 %) de cristaux beiges de 1-bromo 2-(3-fluorophényl) 3-(2-thiénylsulfonyl) 2-propanol. F = 96°C.

## Exemple 54

D'une manière identique à l'exemple 53, 1,8 g (0,006 mole) de 2-(6-chloro 2-pyridyl) 1-(2-thiénylsulfonyl)

2-propène sont traités par deux équivalents de N-bromoacétamide en présence d'une quantité catalytique de perchlorate de lithium dans un mélange acétone/eau/acide trifluoroacétique pendant 2 heures à température ambiante. Le même traitement, suivi d'une chromatographie sur silice du brut (2,4 g) par le mélange éluant chloroforme/heptane : 80/20 donne 1 g (42 %) de cristaux de 1-bromo 2-(6-chloro 2-pyridyl) 3-(2-thiénylsulfonyl) 2-propanol. F = 106°C.

Exemple 55

On dissout 3 g (0,009 mole) de 2-(3,5-dichlorophényl) 1-(2-thiénylsulfonyl) 2-propène dans 25 $cm^3$ d'acétone et refroidit à 0°C. On ajoute successivement 0,52 g d'acétate de tétrabutylammonium, 4,5 $cm^3$ d'une solution aqueuse à 70 % d'hydroperoxyde de ter-butyle et 0,6 g de tétraoxyde d'osmium. Le milieu est agité 45 minutes à 0°C, puis 15 heures à température ambiante. Le milieu est alors refroidi et l'excès d'oxydant est détruit par agitation pendant 1 heure par 25 $cm^3$ d'une solution normale de sulfite de sodium. La phase aqueuse est saturée par du chlorure de sodium et extraite par 3 X 50 $cm^3$ d'éther. Après séchage sur $MgSO_4$ et évaporation, une chromatographie sur silice (éluant : chlorure de méthylène puis acétate d'éthyle) donne 2,2 g (67 %) de cristaux de 2-(3,5-dichlorophényl) 3-(2-thiénylsulfonyl) 1,2-propanediol. F = 104°C.

Exemple 56

1 g (0,0027 mole) de 2-(3,5-dichlorophényl) 3-(2-thiénylsulfonyl) 1,2-propanediol est dissous dans 5 $cm^3$ de pyridine anhydre puis refroidi à 0°C. On ajoute 0,4 g (1,2 équivalent) de chlorure de méthane sulfonyle et agite 2 heures à 0°C, puis 1 heure à température ambiante. Le milieu est versé dans un mélange de 20 g d'eau et de glace et 6 $cm^3$ d'acide chlorhydrique concentré. Après forte agitation, une gomme précipite, qui est filtrée, reprise par 50 $cm^3$ de chloroforme, lavée par 2 X 50 $cm^3$ d'acide chlorhydrique normal et séchée sur $MgSO_4$.

Une chromatographie sur silice (éluant : chlorure de méthylène) du brut (1,4 g de miel) fournit 0,86 g (69 %) de cristaux de 2-(3,5-dichlorophényl) 1-méthylsulfonate 3-(2-thiénylsulfonyl) 2-propanol. F = 66°C.

Exemple 57

1 g (0,0027 mole) de 2-(3,5-dichlorophényl) 3-(2-thiénylsulfonyl) 1,2-propanediol est dissous en mélange avec 0,71 g (0,0027 mole) de triphénylphosphine dans 10 $cm^3$ de chloroforme. On ajoute à 0°C et sous atmosphère inerte, 0,87 g (0,005 mole) de diéthylazodicarboxylate. Le milieu est agité 24 heures à température ambiante, puis concentré sous vide. Le résidu est chromatographié sur silice (éluant : heptane/acétate d'éthyle ; 75/25) pour donner 0,74 g d'huile. Après cristallisation dans un mélange éther/heptane, on filtre 0,47 g (49 %) de cristaux de 2-(3,5-dichlorophényl) 2-[(2-thiénylsulfonyl) méthyl] oxiranne. F = 125°C.

Exemple 58

On dissout 1,4 g (0,0042 mole) de 2-(3,5-dichlorophényl) 1-(2-thiénylsulfonyl) 2-propène dans 15 $cm^3$ de chloroforme et ajoute goutte-à-goutte 0,22 $cm^3$ (1 équivalent) de brome en solution dans 15 $cm^3$ de chloroforme. Après 1 H 30 d'agitation, le solvant est évaporé et le résidu cristallisé dans un mélange éther/chloroforme pour donner 1,2 g (58 %) de cristaux de 2,3-dibromo 2-(3,5-dichlorophényl) 1-(2-thiénylsulfonyl) propane. F = 142°C.

Exemple 59

D'une manière identique à l'exemple 58, on traite 1,8 g (0,005 mole) de 1-[(3-bromo 2-thiényl) sulfonyl] 2-(3-fluorophényl) 2-propène par 1 équivalent de brome dans le chloroforme. Après traitement le résidu huileux (2,6 g) est cristallisé dans l'éther à -18°C pour donner 0,6 g (22 %) de cristaux blancs de 2,3-dibromo 1-[(3-bromo 2-thiényl)sulfonyl] 2-(3-fluorophényl)propane. F = 91°C.

Exemple 60

1,23 g (0,015 mole) d'acétate de sodium sont dissous dans 15 $cm^3$ d'acide acétique. 1,0 g (0,003 mole) de 1-[(3-bromo 2-thiényl) sulfonyl] 2-phényl 2-propène est mis en suspension. On ajoute en une seule fois 0,53 g (0,003 mole) de N-bromosuccinimide à température ambiante et agite 2 heures jusqu'à dissolution totale. Le milieu est versé dans 50 g d'eau glacée et agité jusqu'à précipitation totale. Les cristaux (2,4 g) sont filtrés, séchés et recristallisés dans un mélange chloroforme/éther 3/1 à -18°C, pour donner 0,4 g (30 %) de cristaux

blancs de 2-acétoxy 3-bromo 1-[(3-bromo 2-thiényl)sulfonyl] 2-phényl propane. F = 91°C.

Exemple 61

1,0 g (0,0028 mole) de 1-[(3-bromo 2-thiényl) sulfonyl] 2-(3-fluorophényl) 2-propène est dissous dans 100 $cm^3$ de chloroforme et est refroidi à 0°C. On ajoute goutte-à-goutte 0,5 g (0,03 mole) d'iodure de chlore en solution dans 5 $cm^3$ de chloroforme et agite 2 heures. Le milieu est dilué par 30 $cm^3$ de chloroforme, lavé par 25 $cm^3$ d'une solution à 5 % de bisulfite de sodium, lavé à l'eau et séché sur $MgSO_4$. Le solvant est évaporé sans chauffer, pour donner 1,4 g d'une huile brune. L'huile est dissoute dans 5 $cm^3$ d'éther et mise à -18°C pour donner 0,8 g (55 %) de cristaux bruns de 1-[3-bromo 2-thiényl)sulfonyl] 2-chloro 2-(3-fluorophényl) 3-iodo propane. F = 98°C (dec.).

Les exemples suivants sont préparés selon la méthode de l'exemple 53

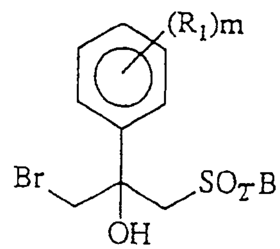

| Exemple | R1 | B | F (Solvant) |
|---|---|---|---|
| 62 | 3,5-diCl | 2-thiényl | 107°C ( C ) |
| 63 | H | 3-Me 2-thiényl | 103°C ( C ) |
| 64 | 3-Cl | 3-thiényl | 103°C ( C ) |
| 65 | 3-Cl 4-F | 3-thiényl | 79°C ( Et2O / CHCl3 ) |
| 66 | 3-Me 4-F | 3-thiényl | 95°C ( Et2O / CHCl3 ) |
| 67 | 3-F | 3-Cl 2-thiényl | 101°C ( Et2O / CHCl3 ) |

C = Chromatographie

Exemple 68

A 0,1 mole d'éthylate de sodium en solution 2 molaire dans l'éthanol, on coule goutte-à-goutte 22,6 g (0,1 mole) d'acétylthiométhylphosphonate de diéthyle en solution dans 20 $cm^3$ d'éthanol à température ambiante. Le milieu est encore agité pendant 1h 30. En maintenant la température à 20°C, on ajoute goutte-à-goutte 30,5 g d'un mélange comprenant 0,1 mole de 1-chloro 2-phényl 2-propène préparé suivant l'exemple 1, en solution dans 30 $cm^3$ d'éthanol. Après 15 mn, le milieu est versé dans 400 $cm^3$ d'eau, réextrait par 3 x 100 $cm^3$ d'éther et les phases éthérées lavées à l'eau et séchées sur $MgSO_4$. L'évaporation laisse 42,1 g d'une huile jaune qui est chromatographiée sur silice (éluant : heptane puis acétate d'éthyle) pour donner 27,4 g (90 %) d'huile jaune de (2-phényl 2-propényl) thiométhylphosphonate de diéthyle. $n_D^{23}$ = 1,5310.

Exemple 69

26 g (0,086 mole) de (2-phényl 2-propényl)thiométhylphosphonate de diéthyle sont dissous dans 500 $cm^3$ de chloroforme et refroidis à 5°C. On ajoute par portions en 30 mn, 44,4 g (0,18 mole) d'acide métachloroperbenzoïque à 70 %. Le milieu est agité 3 heures à température ambiante puis refroidi à 0°C. L'acide métachlorobenzoïque est filtré et la phase organique lavée par 2 x 200 $cm^3$ de soude 0,5N, 2 x 200 $cm^3$ d'eau puis séchée sur $MgSO_4$. L'évaporation laisse 27,3 g d'une huile jaune qui est chromatographiée sur silice (éluant : chloroforme/acétate d'éthyle 9/1) pour donner 19,2 g (67 %) d'huile jaune de (2-phényl 2-propényl)sulfonylméthylphosphonate de diéthyle. $n_D^{23}$ = 1,5250

Exemple 70

5,0 g (0,015 mole) de (2-phényl 2-propényl)sulfonylméthylphosphonate de diéthyle sont dissous dans 45 $cm^3$ de tétrahydrofuranne anhydre et refroidis à -70°C. On ajoute 12 $cm^3$ (0,0165 mole) d'une solution 1,4 molaire de butyllithium dans l'hexane, goutte-à-goutte et laisse agiter 1 heure à -70°C. On ajoute en une seule fois 0,66 g (0,015mole) d'acétaldéhyde et agite 1 heure à -70°C. Le milieu est versé dans 100 $cm^3$ d'eau, réextrait par 3 x 50 $cm^3$ d'éther et les phases éthérées lavées jusqu'à neutralité et séchées sur $MgSO_4$. L'évaporation laisse 2,7 g d'une huile jaune qui est chromatographiée sur silice (éluant : heptane/chloroforme 3/2) pour donner 2,0 g d'huile qui cristallise. Aprés recristallisation dans l'éthanol, on obtient 0,65 g (20 %) de cristaux blancs de (E)-2-phényl 1-(1-propénylsulfonyl)2-propène. F = 56°C.

Les exemples suivants sont préparés suivant la méthode de l'exemple 70.

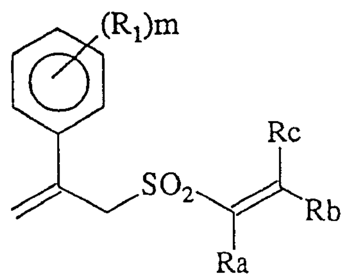

| Exemple | R1 | Ra | Rb | Rc | F (Solvant) nd (Température) |
|---|---|---|---|---|---|
| 71 | 3-F | H | H | H | 1,6910 (24°C) |
| 72 | H | H | C(CH3)3 | H | 56°C ( EtOH ) |
| 73 | H | H | CH3 | CH3 | 61°C ( EtOH ) |
| 74 | H | H | CCl3 | H | 82°C ( EtOH ) |
| 75 | H | H | CH2CH3 | H | 34°C. ( C ) |
| 76 | 3-Me | H | CH3 | H | 1,5619 (21°C) |
| 77 | 3-Me | H | CH2CH3 | H | 39°C ( C ) |
| 78 | 3-Me | H | CH(CH3)2 | H | 1,5461 (21°C) |

C = Chromatographie

L'invention concerne également l'utilisation à titre d'herbicide des composés de formule (I). Comme mauvaises herbes pouvant être contrôlées ou détruites par les composés de formule (I), on peut citer :

Graminées / Cypéracées :

| Abréviations | Nom latin | Nom français |
|---|---|---|
| AVE | Avena fatua | Folle avoine |
| ECH | Echinochloa crusgalli | Panisse |
| LOL | Lolium multiflorum | Ray-grass |
| SOR | Sorghum halepense | Sorgho d'Alep |
| ALO | Alopecurus myosuroïdes | Vulpin |
| CYP | Cyperus esculentus | Cyperus |
| DIG | Digitaria sanguinalis | Digitaire |
| PAN | Panicum miliaceum | Panic |
| SET | Setaria faberie | Millet |

Dicotylédones :

| Abréviations | Nom latin | Nom français |
|---|---|---|
| IPO | Ipomea purpurea | Liseron pourpre |
| SIN | Sinapis arvensis | Moutarde |
| ABU | Abutilon theophrasti | Abutilon |
| SOL | Solanum nigrum | Morelle noire |

L'invention a également pour objet l'utilisation à titre d'herbicide sélectif du riz du 1-[(3-chloro 2-thiényl)sulfonyl] 2-(3-fluorophényl) 2-propène.

L'utilisation des composés de formule (I) est la plupart du temps sous forme de composition herbicide comportant un ou plusieurs supports acceptables en agriculture.

En effet, pour leur emploi pratique, les composés selon l'invention sont rarement utilisés seuls. Le plus souvent ces composés font partie de compositions. Ces compositions, utilisables comme agents herbicides, contiennent comme matière active un composé, selon l'invention, tel que décrit précédemment en mélange avec les supports solides ou liquides, acceptables en agriculture. En particulier, sont utilisables les supports inertes et usuels et les agents tensio-actifs usuels. Ces compositions font également partie de l'invention.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... Plus généralement, les composés utilisés dans l'invention peuvent être combinés à tous les additifs solides ou liquides correspondant aux techniques habituelles de la mise en formulation.

D'une façon générale, les compositions selon l'invention contiennent habituellement de 0,5 à 95 % environ (en poids) d'un composé selon l'invention, un ou plusieurs supports solides ou liquides et éventuellement un ou plusieurs agents tensio-actifs. Par le terme "support", dans le présent exposé, on désigne une matière organique ou minérale, naturelle ou synthétique, avec laquelle le composé est combiné pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau, alcools, notamment le butanol, etc...).

L'agent tensio-actif peut être un agent émulsionnant, dispersant ou mouillant de type ionique ou non ionique ou un mélange de tels agents tensio-actifs. On peut citer, par exemple, des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés, des esters d'acides gras et de polyols, les dérivés à fonction sulfates, sulfonates et phospates des composés précédents. La présence d'au moins un agent tensio-actif est généralement indispensable lorsque le composé et/ou le support inerte ne sont pas solubles dans l'eau et que l'agent vecteur de l'application est l'eau.

Ainsi donc, les compositions à usage agricole, selon l'invention, peuvent contenir les matières actives selon l'invention dans de très larges limites, allant de 0,5 % à 95 % (en poids). Leur teneur en agent tensio-actif est avantageusement comprise entre 5 % et 40 % en poids.

Ces compositions, selon l'invention, sont elles-mêmes sous des formes assez diverses, solides ou liquides.

Comme formes de compositions solides, on peut citer les poudres pour poudrages (à teneur en composé pouvant aller jusqu'à 100 %) et les granulés, notamment ceux obtenus par extrusion, par compactage, par imprégnation d'un support granulé, par granulation à partir d'une poudre (la teneur en composé dans ces granulés étant entre 0,5 et 80 % pour ces derniers cas).

Les poudres mouillables (ou poudres à pulvériser) sont habituellement préparées de manière à ce qu'elles contiennent 20 à 95 % de matière active, et elles contiennent habituellement, en plus du support solide, de 0 à 30 % d'un agent mouillant, de 3 à 20 % d'un agent dispersant, et, quand c'est nécessaire, de 0 à 10 % d'un ou plusieurs stabilisants et/ou autres additifs, comme des agents de pénétration, des adhésifs ou des agents antimottants, colorants, etc...

Pour obtenir les poudres à pulvériser ou poudres mouillables, on mélange intimement les matières actives dans des mélangeurs appropriés avec les substances additionnelles et on broie avec des moulins ou autres broyeurs appropriés. On obtient par là, des poudres à pulvériser dont la mouillabilité et la mise en suspension sont avantageuses, on peut les mettre en suspension avec de l'eau à toute concentration désirée et ces suspensions sont utilisables très avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

A titre d'exemple, voici diverses compositions de poudres mouillables (ou poudres à pulvériser) :

Exemple F1 :

| | |
|---|---|
| - matière active (composé n° 1) | 50,00 % |
| - alcool gras éthoxylé (agent mouillant) | 2,50 % |
| - phényléthylphénol éthoxylé (agent dispersant) | 5,00 % |
| - craie (support inerte) | 42,50 % |

Exemple F2

| | |
|---|---|
| - matière active (composé n° 1) | 10,00 % |
| - Alcool synthétique oxo de type ramifié, en C13 éthoxylé par 8 à 10 oxyde d'éthylène (agent mouillant) | 0,75 % |
| - lignosulfonate de calcium neutre (agent dispersant) | 12,00 % |
| - carbonate de calcium (charge inerte) q.s.p | 100,00 % |

Exemple F3 :

Cette poudre mouillable contient les mêmes ingrédients que dans l'exemple précédent, dans les proportions ci-après :

- matière active 75,00 %
- agent mouillant 1,50 %
- agent dispersant 8,00 %
- carbonate de calcium (charge inerte) q.s.p 100,00 %

Exemple F4

- matière active (composé n° 1) 90,00 %
- alcool gras éthoxylé (agent mouillant) 4,00 %
- phényléthylphénol éthoxylé (agent dispersant) 6,00 %

Exemple F5

- matière active (composé n°1) 50,00 %
- mélange de tensio-actifs anioniques et non ioniques (agent mouillant) 2,50 %
- lignosulfate de sodium (agent dispersant) 5,00 %
- argile kaolinique (support inerte) 42,50 %

Les composés selon l'invention peuvent être formulés sous la forme de granulés dispersibles dans l'eau également compris dans le cadre de l'invention. Ces granulés dispersibles, de densité apparente généralement comprise entre environ 0,3 et 0,6, ont une dimension de particules généralement comprise entre environ 150 et 2000 et de préférence entre 300 et 1500 microns.

La teneur en matière active de ces granulés est généralement comprise entre environ 1 % et 90 %, et de préférence entre 25 % et 90 %.

Le reste du granulé est essentiellement composé d'une charge solide et éventuellement d'adjuvants tensio-actifs conférant au granulé des propriétés de dispersibilité dans l'eau. Ces granulés peuvent être essentiellement de deux types distincts selon que la charge retenue est soluble ou non dans l'eau. Lorsque la charge est hydrosoluble, elle est peut être minérale ou, de préférence, organique. On a obtenu d'excellents résultats avec l'urée. Dans le cas d'une charge insoluble, celle-ci est de préférence minérale, comme par exemple le kaolin ou la bentonite. Elle est alors avantageusement accompagnée d'agents tensioactifs (à raison de 2 à 20 % en poids du granulé) dont plus de la moitié est, par exemple, constituée par au moins un agent dispersant, essentiellement anionique, tel qu'un polynaphtalène sulfonate alcalin ou alcalino-terreux ou un lignosulfonate alcalin ou alcalino-terreux, le reste étant constitué par des mouillants non ioniques ou anioniques tel qu'un alcoyl naphtalène sulfonate alcalin ou alcalino-terreux.

Par ailleurs, bien que cela ne soit pas indispensable, on peut ajouter d'autres adjuvants tels que des agents anti-mousse.

Le granulé selon l'invention peut être préparé par mélange des ingrédients nécessaires puis granulation selon plusieurs techniques en soi connues (drageoir, lit fluide, atomiseur, extrusion, etc...). On termine généralement par un concassagefsuivi d'un tamisage à la dimension de particule choisie dans les limites mentionnées ci-dessus.

De préférence, il est obtenu par extrusion en opérant comme indiqué dans les exemples ci-après.

Exemple F6 : Granulés dispersibles

Dans un mélangeur, on mélange 90 % en poids de matière active (composé n°1) et 10 % d'urée en perles. Le mélange est ensuite broyé dans un broyeur à broches. On obtient une poudre que l'on humidifie avec environ

8 % en poids d'eau. La poudre humide est extrudée dans une extrudeuse à rouleau perforé. On obtient un granulé qui est séché, puis concassé et tamisé, de façon à ne garder respectivement que les granulés d'une dimension comprise entre 150 et 2000 microns.

Exemple F7 : Granulés dispersibles

Dans un mélangeur, on mélange les constituants suivants :

- matière active (composé n° 1) 75,00 %
- agent mouillant (alkylnaphtalène sulfonate de sodium) 2,00 %
- agent dispersant (polynaphtalène sulfonate de sodium) 8,00 %
- charge inerte insoluble dans l'eau (kaolin) 15,00 %

Ce mélange est granulé en lit fluide, en présence d'eau, puis séché, concassé et tamisé de manière à obtenir des granulés de dimension comprise entre 0,15 et 0,80 mm.

Ces granulés peuvent être utilisés seuls, en solution ou dispersion dans de l'eau de manière à obtenir la dose cherchée. Ils peuvent aussi être utilisés pour préparer des associations avec d'autres matières actives, notamment fongicides, ces dernières étant sous la forme de poudres mouillables, ou de granulés ou suspensions aqueuses.

Les composés de formule (I) peuvent encore être utilisés sous forme de poudres pour poudrage, on peut aussi utiliser une composition comprenant 50 g de matière active et 950 g de talc, on peut aussi utiliser une composition comprenant 20 g de matière active, 10 g de silice finement divisée et 970 g de talc, on mélange et broie ces constituants et on applique le mélange par poudrage.

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes.

Les concentrés émulsionnables ou solubles comprennent le plus souvent 10 à 80 % de matière active, les émulsions ou solutions prêtes à l'application contenant, quant à elles, 0,001 à 20 % de matière active.

En plus du solvant, les concentrés émulsionnables peuvent contenir quand c'est nécessaire, 2 à 20 % d'additifs appropriés comme les stabilisants, les agents tensio-actifs, les agents de pénétration les inhibiteurs de corrosion, les colorants ou les adhésifs précédemment cités.

A partir de ces concentrés, on peut obtenir par dilution avec de l'eau des émulsions de toute concentration désirée, qui conviennent particulièrement à l'application sur les cultures. A titre d'exemple, voici la composition de quelques concentrés émulsionnables .

Exemple F8 :

- matière active 400 g/l
- dodécylbenzène sulfonate alcalin 24 g/l
- nonylphénol oxyéthylé à 10 molécules d'oxyde d'éthylène 16 g/l
- cyclohexanone 200 g/l
- solvant aromatique q.s.p 1 litre

Selon une autre formule de concentré émulsionnable, on utilise :

Exemple F9 :

| | |
|---|---|
| - matière active | 250 g |
| - huile végétale époxydée | 25 g |
| - mélange de sulfonate d'alcoylaryle et d'éther de polyglycol et d'alcools gras | 100 g |
| - diméthylformamide | 50 g |
| - xylène | 575 g |

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensio-actifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

A titre d'exemple, voici une composition de suspension concentrée :

Exemple F10 :

| | |
|---|---|
| - composé | 500 g |
| - phosphate de tristyrylphénol polyéthoxylé | 50 g |
| - alkylphénol polyéthoxylé | 50 g |
| - polycarboxylate de sodium | 20 g |
| - éthylène glycole | 50 g |
| - huile organopolysiloxanique (antimousse) | 1 g |
| - polysaccharide | 0,5 g |
| - eau | 316,5 g |

Les dispersions et émulsions aqueuses, par exemple les compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans- l'eau et elles peuvent avoir une consistance épaisse comme celle d'une "mayonnaise".

En ce qui concerne les compositions adaptées au stockage et au transport, elles contiennent plusavantageusement de 0,5 à 95 % (en poids) de substance active.

La présente invention concerne aussi un procédé de désherbage (notamment de zones de culture monocotylédones en particulier maïs, blé, orge, riz) qui consiste à appliquer aux plantes devant être détruites une quantité efficace d'un composé de formule (I).

Lors de l'application à une zone cultivée, la dose d'application devrait être suffisante pour contrôler la naissance des adventices sans causer de dommages substantiels permanents auxdites cultures. Par dose efficace, on entend justement dans ce contexte, la dose qui permet d'obtenir ce résultat.

Les produits et compositions selon l'invention s'appliquent de préférence sur les mauvaises herbes à éliminer lorsque celles-ci présentent un feuillage vert, avantageusement les dicotylédones.

Néanmoins, on pourra utiliser également un procédé de désherbage qui consiste à appliquer une quantité efficace d'un composé de formule (I) sur des zones ou terrains où l'on veut empêcher la pousse ou le développement de plantes n'ayant pas encore poussé (application de préemergence).

On peut opérer de manière à ce que la culture soit semée avant ou après traitement.

L'invention a également pour objet un procédé de désherbage du riz en post repiquage pour contrôler les adventices en pré ou post levée à l'aide du composé n° 5 qui est le 1-[(3-chloro 2-thiényl)sulfonyl] 2-(3-fluorophényl) 2-propène.

La dose d'application de matière active est généralement comprise entre 1 et 8000 g/ha.

Les exemples ci-dessous illustrent l'invention :

Exemple A - Application herbicide, en prélevée des espèces végétales

Dans des pots de 7 x 7 x 8 cm remplis de terre agricole légère, on sème un nombre de graines déterminé en fonction de l'espèce végétale et de la grosseur de la graine.

Les pots sont traités par pulvérisation de bouillie en quantité correspondant à une dose volumique d'application de 500 l/ha et contenant la matière active à la concentration désirée.

Le traitement avec la bouillie est donc effectué sur des graines non recouvertes de terre (on utilise le terme de bouillie pour désigner, en général, les compositions diluées à l'eau, telles qu'on les applique sur les végétaux).

La bouillie utilisée pour le traitement est une solution ou suspension de la matière active dans un mélange acétone/eau en proportions 50/50, en présence de 0,05 % en poids de cemulsol NP 10 (agent tensio-actif) constitué d'alkylphénol polyéthoxylé, notamment de nonylphénol polyéthoxylé) et 0,04 % en poids de tween 20 (agent tensio-actif constitué d'un oléate de dérivé polyoxyéthyléné du sorbitol).

Dans le cas d'une suspension, celle-ci est obtenue en mélangeant et broyant les ingrédients dans un microniseur de façon à obtenir une grosseur moyenne de particules inférieure à 40 microns.

Après traitement, on recouvre les graines d'une couche de terre d'environ 3 mm d'épaisseur.

Les pots sont alors placés dans des bacs destinés à recevoir l'eau d'arrosage, en subirrigation, et maintenus pendant 24 jours à température ambiante sous 70 % d'humidité relative.

Notation de l'activité herbicide

Le relevé est effectué de la façon suivante :

Au bout de 24 jours, on mesure un pourcentage (D) des destructions du nombre de pieds dans le pot traité par rapport au nombre de plantes dans les pots non traités (témoin). On mesure, sur les plantes traitées restantes, le pourcentage de réduction de taille (RT) par rapport aux plantes témoin.

Le pourcentage du volume foliaire non détruit par le produit est donc donné par la formule :

$$\frac{[100-D] \times [100-RT]}{100} = A$$

Cette valeur A est transformée en notation de 0 à 5 selon l'échelle suivante :

| | Notation |
|---|---|
| 0 à 10 | 5 (destruction complète) |
| 10 à 30 | 4 |
| 30 à 50 | 3 |
| 50 à 70 | 2 |
| 70 à 90 | 1 |
| 90 à 100 | 0. (pas d'effet) |

Les résultats obtenus sont présentés après l'exemple B pour des doses d'application de 4000 g/ha à 1000 g/ha.

Exemple B - Application herbicide, en postlevée des espèces végétales

Dans des pots de 7 x 7 x 8 cm remplis de terre agricole légère, on sème un nombre de graines déterminé en fonction de l'espèce végétale et de la grosseur de la graine.

On recouvre ensuite les graines d'une couche de terre d'environ 3 mm d'épaisseur et on laisse germer la graine jusqu'à ce qu'elle donne naissance à une plantule au stade convenable. Le stade de traitement pour les graminées est le stade "deuxième feuille en formation ". Le stade de traitement pour les dicotylédones, est le stade "cotylédons étalés, première feuille vraie en développement".

Les pots sont alors traités par pulvérisation de bouillie en quantité correspondant à une dose volumique d'application de 500 l/ha et contenant la matière active à la concentration désirée.

La bouille a été préparée de la même manière qu'à l'exemple A.

Les pots traités sont ensuite placés dans des bacs destinés à recevoir l'eau d'arrosage, en subirrigation, et maintenus pendant 24 jours à température ambiante sous 70 % d'humidité relative.

Notation de l'activité herbicide

Le relevé est effectué de la façon suivante :

Au bout de 24 jours, on mesure un pourcentage (D) de destruction du nombre de pieds dans le pot traité par rapport au nombre de plantes dans les pots non traités (témoin). On mesure, sur les plantes traitées restantes, le pourcentage de réduction de taille (RT) par rapport aux plantes témoin.

Le pourcentage du volume foliaire non détruit par le produit est donc donné par la formule :

$$\frac{[100-D] \times [100-RT]}{100} = A$$

Cette valeur A est transformée en notation de 0 à 5 selon l'échelle suivante :

| | Notation |
|---|---|
| 0 à 10 | 5 (destruction complète) |
| 10 à 30 | 4 |
| 30 à 50 | 3 |
| 50 à 70 | 2 |
| 70 à 90 | 1 |
| 90 à 100 | 0 (pas d'effet) |

Les résultats obtenus sont présentés après le tableau A pour des doses d'application de 4000 g/ha à 1000 g/ha.

Les espèces végétales utilisées dans ces exemples A et B sont :

| ABREVIATIONS | NOM LATIN | NOM FRANCAIS |
|---|---|---|
| AVE | Avena fatua | Folle avoine |
| ALO | Alopecurus myosuroïdes | Vulpin |
| ECH | Echinochloa crus galli | Panisse |
| CYP | Cyperus esculentus | Cyperus |
| DIG | Digitaria sanguinalis | Digitaire |

## ACTIVITE HERBICIDE DE PRELEVEE

| Composé N° | Dose en Kg m.a./ha | AVE | ECH | DIG | CYP | ALO |
|---|---|---|---|---|---|---|
| 3 | 1 | 1 | 5 | 5 | 5 | 5 |
| 4 | 4 | 3 | 5 | 5 | 5 | 5 |
| 5 | 4 | 5 | 5 | 5 | 5 | 5 |
| 7 | 1 | 1 | 5 | 5 | 1 | 5 |
| 9 | 4 | 5 | 5 | 5 | 4 | 5 |
| 10 | 4 | 5 | 5 | 5 | 0 | |
| 11 | 4 | 5 | 5 | 5 | 3 | 5 |
| 12 | 4 | 2 | 5 | 5 | 0 | 5 |
| 13 | 1 | 5 | 5 | 5 | 1 | 3 |
| 15 | 4 | 2 | 5 | 5 | 2 | 4 |
| 17 | 1 | 1 | 5 | 5 | 3 | 1 |
| 19 | 4 | 1 | 5 | 5 | 0 | 5 |
| 20 | 1 | 4 | 5 | 4 | 1 | 3 |
| 21 | 1 | 0 | 5 | 5 | 0 | 2 |
| 22 | 4 | 5 | | 5 | 1 | 5 |
| 24 | 1 | 1 | 5 | 5 | 0 | 4 |
| 25 | 4 | 5 | | 5 | 1 | 2 |
| 26 | 4 | 5 | | 5 | 5 | 5 |
| 27 | 1 | 0 | 4 | 4 | 0 | 2 |
| 28 | 4 | 2 | | 5 | 0 | 5 |
| 29 | 4 | 5 | | 5 | 5 | 5 |
| 30 | 1 | 2 | 5 | 5 | 0 | 2 |
| 31 | 4 | 5 | 5 | 5 | 4 | 5 |
| 32 | 1 | 2 | 4 | 5 | 0 | 5 |
| 33 | 4 | 1 | 4 | 4 | 4 | 4 |
| 34 | 1 | 3 | 5 | 5 | 4 | 3 |
| 35 | 1 | 2 | 5 | 5 | 1 | 5 |
| 36 | 4 | 3 | 5 | 5 | 1 | 5 |

| Composé N° | Dose en Kg m.a./ha | AVE | ECH | DIG | CYP | ALO |
|---|---|---|---|---|---|---|
| 37 | 2 | 4 | 5 | | 1 | 5 |
| 39 | 4 | 4 | 5 | | 0 | 5 |
| 40 | 2 | 2 | 4 | | 1 | 3 |
| 42 | 2 | 3 | 5 | 5 | 1 | 5 |
| 43 | 2 | 5 | 5 | | 2 | 5 |
| 44 | 4 | 5 | 5 | | 5 | 5 |
| 45 | 2 | 3 | 5 | 5 | 3 | 5 |
| 46 | 2 | 4 | 5 | 5 | 1 | 5 |
| 47 | 2 | 5 | 5 | 5 | 0 | 5 |
| 51 | 4 | 4 | 5 | 5 | 1 | 5 |
| 53 | 1 | 1 | 5 | 5 | 4 | 2 |
| 54 | 1 | 1 | 5 | 5 | 5 | 5 |
| 56 | 1 | 5 | 5 | 5 | 2 | 5 |
| 60 | 2 | 4 | 5 | | 4 | 3 |
| 62 | 1 | 4 | 2 | 5 | 1 | 2 |
| 63 | 1 | 2 | 5 | 5 | 0 | 5 |
| 64 | 1 | 4 | | 5 | 5 | 4 |
| 65 | 1 | 3 | 5 | 5 | 1 | 2 |
| 66 | 1 | 5 | 5 | 5 | 1 | 2 |
| 67 | 1 | 4 | 5 | 5 | 2 | 3 |
| 70 | 4 | 4 | 5 | | 2 | 5 |
| 72 | 4 | 3 | 3 | 4 | 0 | 5 |
| 73 | 2 | 2 | 5 | 5 | 0 | 5 |
| 74 | 4 | 0 | 4 | 5 | 0 | 3 |

ACTIVITE HERBICIDE DE POSTLEVEE

| Compose N° | Dose en Kg m.a./ha | ECH | DIG | ALO |
|---|---|---|---|---|
| 5 | 4 | | 3 | 4 |
| 7 | 4 | 4 | 3 | 4 |
| 9 | 4 | 3 | 4 | 3 |
| 17 | 4 | 4 | 4 | 5 |
| 18 | 4 | 4 | 3 | 4 |
| 20 | 1 | 4 | 0 | 2 |
| 22 | 4 | | 3 | 4 |
| 26 | 4 | 5 | 3 | 4 |
| 28 | 4 | 4 | 0 | 4 |
| 29 | 4 | 5 | 1 | 5 |
| 32 | 4 | 3 | 3 | 2 |
| 35 | 4 | 4 | 3 | 4 |
| 37 | 2 | 3 | | |
| 39 | 4 | 3 | 3 | |
| 40 | 2 | 3 | | |
| 42 | 2 | 2 | 4 | |
| 44 | 1 | 3 | 4 | |
| 45 | 2 | 3 | 1 | |
| 46 | 2 | 3 | 3 | |
| 47 | 2 | 3 | 1 | |
| 51 | 4 | 3 | 4 | |
| 53 | 1 | 3 | 4 | 1 |
| 60 | 2 | 4 | | |
| 63 | 1 | 4 | 4 | 1 |
| 66 | 4 | 0 | 4 | 4 |
| 70 | 4 | 3 | | |
| 73 | 2 | 0 | 4 | |
| 74 | 4 | 0 | 4 | |

<u>Exemple C</u> - <u>Essai de sélectivité en grandes cultures avec application herbicide, en prélevée des espèces végétales</u>

Dans des pots de 7 X 7 X 8 cm remplis de terre agricole légère, on sème un nombre de graines déterminé en fonction de l'espèce végétale et de la grosseur de la graine.

On recouvre ensuite les graines d'une couche de terre d'environ 3 mm d'épaisseur.

Les pots sont alors traités par pulvérisation de bouillie en quantité correspondant à une dose volumique d'application de 500 l/ha et contenant la matière active à la concentration désirée.

La bouillie a été préparée de la même manière qu'à l'exemple A.

Les pots traités sont ensuite placés dans des bacs destinés à recevoir l'eau d'arrosage, en subirrigation, et maintenus pendant 24 jours à température ambiante sous 70 % d'humidité relative.

Notation de l'activité herbicide

Le relevé est effectué de la façon suivante :

Au bout de 24 jours, on mesure un pourcentage (D) de destruction du nombre de pieds dans le pot traité par rapport au nombre de plantes dans les pots non traités (témoin). On mesure, sur les plantes traitées restantes, le pourcentage de réduction de taille (RT) par rapport aux plantes témoins.

Le pourcentage de volume foliaire non détruit par le produit est donc donné par la formule :

$$\frac{[100-D] \times [100-RT]}{100} = A$$

Cette valeur A est transformée en notation de 0 à 5 selon l'échelle suivante :

| | | | notation | |
|---|---|---|---|---|
| 0 | à | 10 | 5 | (destruction complète) |
| 10 | à | 30 | 4 | |
| 30 | à | 50 | 3 | |
| 50 | à | 70 | 2 | |
| 70 | à | 90 | 1 | |
| 90 | à | 100 | 0 | (pas d'effet) |

Ainsi, un produit est jugé comme sélectif vis à vis de la culture lorsque la valeur A notée est de 0 ou 1.

Les résultats obtenus sont présentés dans l'exemple C pour des doses d'application de 500, 1000, 2000 ou 4000 g de matière active par hectare selon les produits.

Les espèces végétales utilisées dans cet exemple sont :

1) Pour les adventices

| ABREVIATIONS | NOM LATIN | NOM FRANCAIS |
|---|---|---|
| ECH | Echinochloa crus-galli | Panisse |
| PAN | Panicum milicaeum | Panic |
| DIG | Digitaria sanguinalis | Digitaire |
| SOR | Sorghum halepense | Sorgho d'Alep |
| SET | Setaria faberii | Millet |
| CYP | Cyperus esculentus | Cyperus |
| ALO | Alopecurus myosuroides | Vulpin |

2) Pour les cultures

| ABREVIATIONS | NOM LATIN | NOM FRANCAIS |
| --- | --- | --- |
| TRZ | Triticum aestivum | Blé |
| ZEA | Zea mays | Maïs |
| ORY | Oryza sativa | Riz |
| GLX | Glycine maximum | Soja |

ESSAI DE SELECTIVITE EN GRANDES CULTURES
ACTIVITE HERBICIDE DE PRELEVEE

| Composé N° | Dose en Kg m.a./ha | ECh | PAN | DIG | SCR | SET | ALO | TRZ | ZEA | CRY | GLY |
|---|---|---|---|---|---|---|---|---|---|---|---|
| 3 | 1 | 5 | 5 | 5 | 5 | 3 | 5 | 2 | 0 | 0 | 3 |
| 4 | 2 | | 2 | | 5 | 3 | 5 | 1 | 1 | 1 | 0 |
| 5 | 0.5 | 4 | 2 | 5 | 3 | 5 | 2 | 0 | 0 | 0 | 0 |
| 7 | 1 | 5 | 3 | 5 | 2 | 5 | 2 | 2 | 0 | 0 | 0 |
| 9 | 1 | 4 | 4 | 5 | 5 | 4 | 1 | 0 | 0 | 0 | 0 |
| 11 | 2 | 4 | 2 | 5 | 4 | 2 | | 0 | 1 | 0 | 0 |
| 13 | 2 | 5 | 5 | 5 | 3 | 5 | | 2 | 0 | 2 | 0 |
| 14 | 2 | 5 | 5 | 5 | 5 | 4 | 5 | 1 | 1 | 1 | 0 |
| 15 | 4 | 5 | 3 | 5 | 0 | 3 | | 0 | 0 | 0 | 0 |
| 17 | 1 | | 5 | 5 | 5 | 5 | 3 | 0 | 0 | 0 | 0 |
| 18 | 0.5 | 4 | 3 | 5 | 2 | 3 | 0 | 1 | 0 | 0 | 0 |
| 21 | 2 | 5 | 5 | 5 | 5 | 3 | 5 | 1 | 0 | 3 | 0 |
| 22 | 1 | 5 | 5 | 5 | 0 | 0 | 5 | 0 | 0 | 1 | 0 |
| 23 | 2 | | 5 | 5 | 0 | 1 | 2 | 0 | 0 | 0 | 0 |
| 24 | 1 | | 3 | 5 | 5 | 2 | 3 | 0 | 0 | 0 | 0 |
| 25 | 1 | 5 | 0 | 5 | 4 | 4 | 5 | 0 | 0 | 5 | 0 |
| 27 | 1 | 4 | 3 | 5 | 0 | 2 | 3 | 0 | 0 | 0 | 0 |
| 30 | 1 | 5 | 3 | 5 | 2 | 2 | 3 | 1 | 0 | 0 | 0 |
| 31 | 2 | 5 | 5 | 5 | 3 | 3 | | 2 | 0 | 1 | 0 |
| 34 | 2 | 5 | 4 | 5 | 2 | 5 | 0 | 1 | 0 | 1 | 0 |
| 35 | 2 | 5 | 5 | 5 | 5 | 4 | 4 | 2 | 0 | 1 | 0 |
| 36 | 1 | 5 | 5 | 5 | 3 | 4 | 2 | 0 | 0 | 0 | 0 |
| 37 | 0.5 | 5 | 5 | | 4 | 3 | 1 | 0 | 0 | 0 | 0 |
| 38 | 1 | 5 | 3 | 5 | 5 | 5 | 3 | 0 | 0 | 0 | 0 |
| 39 | 0.5 | 5 | 5 | 5 | 3 | 4 | 4 | 0 | 0 | 0 | 0 |
| 40 | 2 | 5 | 5 | | 5 | 4 | 5 | 0 | 0 | 1 | 0 |
| 42 | 2 | 5 | 5 | 4 | 4 | 3 | 4 | 1 | 0 | 1 | 0 |
| 43 | 2 | 3 | 5 | | 5 | 5 | 5 | 2 | 0 | 2 | 0 |
| 44 | 1 | 5 | 5 | 5 | 3 | 4 | 5 | 0 | 0 | 0 | 0 |

| Composé N° | Dose en Kg m.a./ha | ECh | PAN | DIG | SCR | SET | ALO | TRZ | ZEA | ORY | GLY |
|---|---|---|---|---|---|---|---|---|---|---|---|
| 45 | 1 | 5 | 5 | 4 | 5 | 3 | 5 | 1 | 0 | 0 | 0 |
| 46 | 1 | 4 | 5 | 5 | 1 | 1 | 5 | 0 | 0 | 0 | 0 |
| 47 | 1 | 4 | 5 | 5 | 3 | 2 | 5 | 0 | 1 | 2 | 0 |
| 51 | 1 | 4 | 4 | 5 | 4 | 4 | 4 | 0 | 0 | 0 | 0 |
| 53 | 0.5 | 4 | 1 | 4 | 4 | 1 | 2 | 0 | 2 | 3 | 0 |
| 56 | 1 | 5 | 5 | 5 | 2 | 5 | | 2 | 1 | 2 | 0 |
| 60 | 0.5 | 5 | 3 | | 5 | 4 | 2 | 0 | 0 | 0 | 0 |
| 62 | 1 | 5 | 4 | 5 | 2 | 4 | | 2 | 1 | 2 | 0 |
| 63 | 1 | | 4 | 5 | 5 | 5 | 3 | 3 | 2 | 4 | 0 |
| 64 | 1 | 5 | 5 | 5 | 2 | 5 | 3 | 0 | 2 | 2 | 0 |
| 65 | 0.5 | 5 | 3 | 5 | 5 | 0 | 0 | 0 | 0 | 3 | 0 |
| 66 | 1 | 5 | 3 | 5 | 5 | 3 | 3 | 1 | 2 | 2 | 1 |
| 67 | 0.5 | 4 | 4 | 5 | 1 | 4 | 2 | 0 | 1 | 0 | 0 |
| 70 | 2 | 5 | 3 | | 5 | 3 | 4 | 2 | 0 | 1 | 0 |
| 73 | 2 | 5 | 3 | 3 | 3 | 0 | 4 | 1 | 2 | 0 | 0 |

Comme on peut le voir sur le tableau de résultats de cet exemple C, de nombreux produits présentent une excellente activité antigraminée de prélevée tout en montrant une excellente sélectivité pour 1 ou 2 ou 3 ou 4 des 4 cultures testées = blé, maïs, riz, soja.

Exemple D : Application herbicide en pré ou post levée d'espèces végétales submergées (modèle rizière)

Dans des pots de 9 x 9 x 9 cm remplis de terre agricole on sème un nombre de graines ou on plante un nombre de bulbes ou rhizomes déterminé en fonction de l'espèce végétale ou de la grosseur des organes. Pour le riz, 4 plants au stade 2-3 feuilles sont transplantés dans un pot rempli de terre végétale 4 jours avant le traitement.

Les pots sont ensuite immergés dans un bac de taille adapté aux pots de manière à ce que l'eau recouvre de 1 à 2 cm la surface du sol.

Le stade de traitement est le stade prélevée ou 1 à 2 feuilles développées des adventices. Le traitement est effectué par répartition de la bouillie dans l'eau de submersion en quantité correspondant à une dose d'application de 500 l/ha et contenant la matière active à la concentration requise.

La bouillie utilisée pour le traitement est une solution ou suspension de la matière active dans un mélange acétone/eau en proportion 50/50, en présence de 0,05 % en poids de Cemulsol NP 10 (agent tensioactif constitué d'alkylphénol polyéthoxylé, notamment de nonylphénol polyéthoxylé) et 0,04 % en poids de tween 20 (agent tensio-actif constitué d'un oléate de dérivé polyoxyéthyléné du sorbitol).

Dans le cas d'une suspension, celle-ci est obtenue en mélangeant et broyant les ingrédients dans un microniseur de façon à obtenir une grosseur moyenne de particules inférieures à 40 microns.

Le niveau d'eau de submersion est ensuite maintenu par apport d'eau tout au long de la durée de l'expérimentation soit 28 jours. Les bacs contenant les pots sont placés dans une serre régulée en température à 18°C la nuit et 22°C le jour et en humidité à 70 % d'humidité relative.

Notation de l'activité herbicide

Au bout de 24 jours, on compte le nombre de plantes vivantes dans les pots traités par la bouillie contenant

la matière active à tester et le nombre de plantes vivantes dans un pot témoin traité selon les mêmes conditions, mais au moyen d'une bouillie ne contenant pas de matière active. On mesure ainsi un pourcentage (D) des destructions du nombre de pieds dans le pot traité par rapport au nombre de plantes dans les pots non traités (témoin). On mesure, sur les plantes traitées restantes, le pourcentage de réduction de taille (RT) par rapport aux plantes témoin.

Le pourcentage du volume foliaire non détruit par le produit est donc donné par la formule :

$$\frac{[100-D] \times [100-RT]}{100} = A$$

Cette valeur A est transformée en notation de 0 à 5 selon l'échelle suivante :

| | Notation |
|---|---|
| 0 à 10 | 5 (destruction complète) |
| 10 à 30 | 4 |
| 30 à 50 | 3 |
| 50 à 70 | 2 |
| 70 à 90 | 1 |
| 90 à 100 | 0 (pas d'effet) |

Les résultats obtenus sont présentés dans le tableau suivant pour des doses d'application de 500, 1000 ou 2000 g de matière active par hectare.

EXEMPLE :

COMPOSE N°5/RIZ (transplanté 4 jours avant traitement)

| | Composé N°5 | | | Butachlor | |
|---|---|---|---|---|---|
| % d'efficacité | 500 | 1000 | 2000 | 500 | 1000 |
| ORYSA SATIVA Cv Nihonbare | 0 | 0 | 0 | 0 | 0 |
| ECHINOCHLOA CRUS GALLI (prélevée) | 5 | 5 | 5 | 5 | 5 |
| ECHINOCHLOA CRUS GALLI (1 feuille | 4 | 4 | 4 | 1 | 4 |
| ALISMA CANALICULATUM (2 feuilles) | 3 | 4 | 4 | 4 | 5 |
| CYPERUS SEROTINUS (prélevée) | 2 | 5 | 5 | 0 | 1 |
| CYPERUS SEROTINUS (1 feuille) | 3 | 4 | 5 | 0 | 1 |

## Revendications

1. Composés de formule :

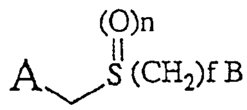

(I)

dans laquelle :

n = 0, 1, 2

f = 0, 1

A est choisi parmi les groupes

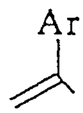

($A_1$)

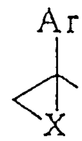

($A_2$)

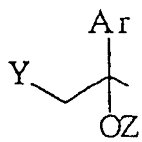

($A_3$)

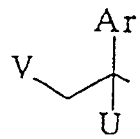

($A_4$)

dans lesquels :

Ar est choisi parmi les groupes

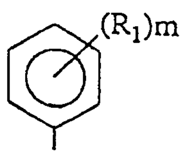

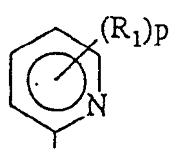

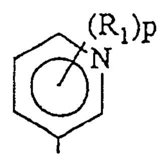

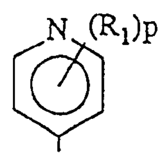

Ar-1 Ar-2 Ar-3 Ar-4

X étant un atome d'oxygène ou de soufre,

$R_1$ étant un atome d'halogène (notamment Cl ou Br ou F), un groupe $C_1$-$C_4$ alkyle, $C_1$-$C_4$ alkoxy, $C_1$-$C_4$ haloalkyle, $C_1$-$C_4$ haloalkoxy, nitro, cyano, $C_6$-$C_{10}$ aryle (notamment phényle ou naphtyle), $C_7$-$C_{11}$ aralkyle (notamment benzyle), $C_6$-$C_{10}$ aryloxy (notamment phénoxy ou naphthoxy) éventuellement substitué par 1 ou 2 atomes d'halogène, $C_7$-$C_{11}$ aralkyloxy (notamment benzyloxy) éventuellement substitué par 1 ou 2 atomes d'halogène,

m = 0, 1, 2, 3, 4, 5,

p = 0, 1, 2, 3, 4,

les différents radicaux $R_1$ étant identiques ou différents lorsque m ou p est supérieur ou égal à 2,

Y est un atome de chlore ou de brome ou d'iode ou un groupement $OSO_2R_2$,

$R_2$ étant un groupement $C_1$-$C_8$ alkyle, $C_6$-$C_{10}$ aryle, $C_7$-$C_{11}$ aralkyle, les dits groupements étant éventuellement substitués par 1 à 3 halogènes ou groupements $C_1$-$C_4$ alkyle, $C_1$-$C_4$ alkoxy, $C_1$-$C_4$ haloalkyle, $C_1$-$C_4$ haloalkoxy, nitro,

Z étant un atome d'hydrogène ou un groupe (C=O)$R_5$,

$R_5$ est hydrogène, $C_1$-$C_4$ alkyle, $C_1$-$C_4$ haloalkyle,

U étant un atome de brome ou de chlore,

V étant un atome de chlore, de brome, ou d'iode,

B est choisi parmi les groupes $C_2$-$C_{10}$ alcényle, ces groupes étant éventuellement substitués par 1 à 6 atomes d'halogéne ou choisi parmi les groupes

B1 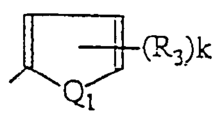 B2 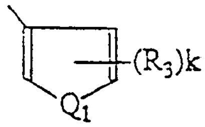

B3 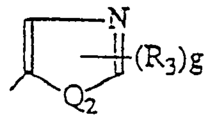 B4 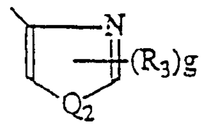 B5 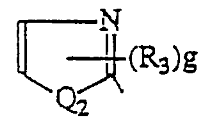

B6 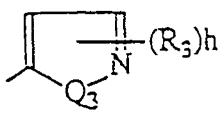 B7 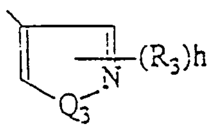 B8 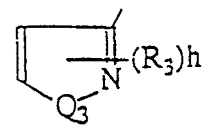

$R_3$ ayant l'une des significations indiquées pour $R_1$ ou le groupe $C_1$-$C_4$ thioalkyl, les différents radicaux $R_3$ étant identiques ou différents lorsque k ou g ou h est supérieur ou égal à 2,
$Q_1$ étant un atome de soufre, d'oxygène ou le groupe $NR_4$,
$Q_2$ étant un atome de soufre ou d'oxygène,
$Q_3$ étant un atome de soufre ou d'oxygène,
$R_4$ est hydrogène, $C_1$-$C_4$ alkyle, $C_1$-$C_4$ haloalkyle,
k = 0, 1, 2, 3,
g = 0, 1, 2,
h = 0, 1, 2.
à l'exception des composés de formule (I) où A est $A_1$ avec $A_r$ est phényle et B est 1-isopropyl 2-méthyl 1-propenyle, n = 0, f = 0 ou B est vinyle, n = 2, f = 0 ou B est 3,3,3 trifluoro 2-trifluorométhyl 1-propényle, n = 0, f = 0.

**2.** Composé selon la revendication 1, caractérisé en ce que n = 2.

**3.** Composé selon la revendication 1, caractérisé en ce que X= O

**4.** Composé selon la revendication 1, caractérisé en ce que m est inférieur ou égal à 2.

**5.** Composé selon la revendication 1, caractérisé en ce que p est inférieur ou égal à 2.

**6.** Composé selon la revendication 1, caractérisé en ce que k est inférieur ou égal à 2.

**7.** Composé selon la revendication 1, caractérisé en ce que $R_1$ est halogène, nitro, trifluorométhyle, méthoxy, méthyle, éthyle.

**8.** Composé selon la revendication 1, caractérisé en ce que Z est hydrogène.

**9.** Composé selon la revendication 1, caractérisé en ce que U et V sont l'atome de brome.

**10.** Utilisation des composés selon l'une des revendications 1 à 10 à titre d'herbicide notamment sous la forme d'une composition herbicide comportant en outre un support inerte.

**11.** Composition herbicide comportant 0,5 à 95 % en poids d'une matière active selon l'une des revendications 1 à 10 en combinaison avec les supports solides ou liquides acceptables en agriculture et les agents tensio-actif acceptables en agriculture.

**12.** Procédé de désherbage (notamment de zones de culture dicotylédone ou de maïs) qui consiste à appliquer aux plantes devant être détruites une quantité efficace d'un composé de formule (I) selon la revendication 1.

13. Procédé de désherbage (notamment de zones de culture dicotylédone ou de maïs) qui consiste à appliquer aux plantes devant être détruites une quantité efficace d'un composé de formule (I) selon la revendication 1 en pré-émergence.

14. Procédé de désherbage des rizières où le riz a été post repiqué par application d'une quantité efficace de 1-[(3-chloro 2-thiényl) sulfonyl] 2-[3-fluorophényl) 2-propène

15. Composés de formule (II) à (XV) dans lesquelles les différents substituants ont la même signification que dans la revendication 1 utiles notamment comme intermédiaires dans les procédés de préparation des composés de formule (I).

**Revendications pour l'Etat contractant suivant : GR**

1. Composition herbicide contenant un ou plusieurs composés de formule :

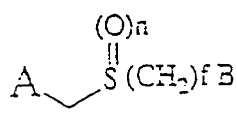 (I)

dans laquelle :
n = 0, 1, 2
f = 0, 1
A est choisi parmi les groupes

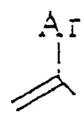 ($A_1$) 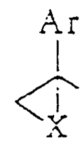 ($A_2$)

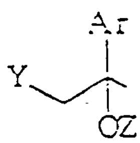 ($A_3$) 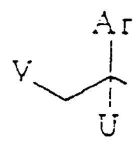 ($A_4$)

dans lesquels :
Ar est choisi parmi les groupes

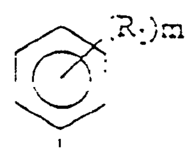 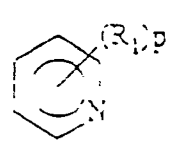 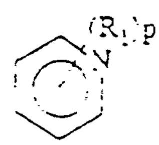 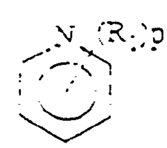

Ar-1 Ar-2 Ar-3 Ar-4

X étant un atome d'oxygène ou de soufre,
$R_1$ étant un atome d'halogène (notamment Cl ou Br ou F), un goupe $C_1$-$C_4$ alkyle, $C_1$-$C_4$ alkoxy, $C_1$-$C_4$ haloalkyle, $C_1$-$C_4$ haloalkoxy, nitro, cyano, $C_6$-$C_{10}$ aryle (notamment phényle ou naphtyle), $C_7$-$C_{11}$ aralkyle (notamment benzyle), $C_6$-$C_{10}$ aryloxy (notamment phénoxy ou naphthoxy) éventuellement substitué par 1 ou 2 atomes d'halogène, $C_7$-$C_{11}$ aralkyloxy (notamment benzyloxy) éventuellement substitué par 1 ou 2 atomes d'halogène,
m = 0, 1, 2, 3, 4, 5,
p = 0, 1, 2, 3, 4,
les différents radicaux $R_1$ étant identiques ou différents lorsque m ou p est supérieur ou égal à 2, Y

est un atome de chlore ou de brome ou d'iode ou un groupement $OSO_2R_2$,
$R_2$ étant un groupement $C_1$-$C_6$ alkyle, $C_6$-$C_{10}$ aryle, $C_7$-$C_{11}$ aralkyle, les dits groupements étant éventuellement substitués par 1 à 3 halogènes ou groupements $C_1$-$C_4$ alkyle, $C_1$-$C_4$ alkoxy, $C_1$-$C_4$ haloalkyle, $C_1$-$C_4$ haloalkoxy, nitro,
Z étant un atome d'hydrogène ou un groupe
$(C{=}O)R_5$,
$R_5$ est hydrogène, $C_1$-$C_4$ alkyle, $C_1$-$C_4$ haloalkyle,
U étant un atome de brome ou de chlore,
V étant un atome de chlore, de brome ou d'iode,
B est choisi parmi les groupes $C_2$-$C_{10}$ alcényle, ces groupes étant éventuellement substitués par 1 à 6 atomes d'halogène ou choisi parmi les groupes

B1 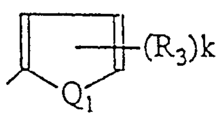 B2 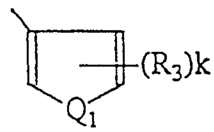

B3 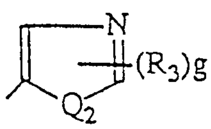 B4 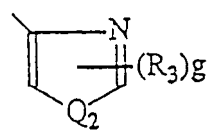 B5 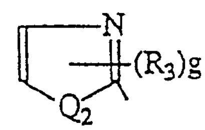

B6 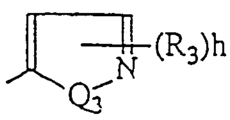 B7 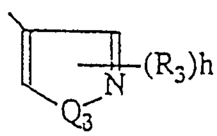 B8 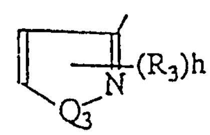

$R_3$ ayant l'une des significations indiquées pour $R_1$ ou le groupe $C_1$-$C_4$ thioalkyl, les différents radicaux $R_3$ étant identiques ou différents lorsque k ou g ou h est supérieur ou égal à 2,
$Q_1$ étant un atome de soufre, d'oxygène ou le groupe $NR_4$,
$Q_2$ étant un atome de soufre ou d'oxygène,
$Q_3$ étant un atome de soufre ou d'oxygène,
$R_4$ est hydrogène, $C_1$-$C_4$ alkyle, $C_1$-$C_4$ haloalkyle,
k = 0, 1, 2, 3,
g = 0, 1, 2,
h = 0, 1, 2.
à l'exception des composés de formule (I) où A est $A_1$ avec $A_r$ est phényle et B est 1-isopropyl 2-méthyl 1-propenyle, n = 0, f = 0 ou B est vinyle, n = 2, f = 0 ou B est 3,3,3 trifluoro 2-trifluorométhyl 1-propényle, n = 0, f = 0.

2. Composition selon la revendication 1, caractérisée en ce que n = 2.

3. Composition selon la revendication 1, caractérisée en ce que X = O.

4. Composition selon la revendication 1, caractérisée en ce que m est inférieur ou égal à 2.

5. Composition selon la revendication 1, caractérisée en ce que p est inférieur ou égal à 2.

6. Composition selon la revendication 1, caractérisée en ce que k est inférieur ou égal à 2.

7. Composition selon la revendication 1, caractérisée en ce que $R_1$ est halogène, nitro, trifluorométhyle, méthoxy, méthyle, éthyle.

8. Composition selon la revendication 1, caractérisée en ce que Z est hydrogène.

**9.** Composition selon la revendication 1, caractérisée en ce que U et V sont l'atome de brome.

**10.** Utilisation des composés selon l'une des revendications 1 à 10 à titre d'herbicide notamment sous la forme d'une composition herbicide comportant en outre un support inerte.

**11.** Composition herbicide comportant 0,5 à 95 % en poids d'une matière active selon l'une des revendications 1 à 10 en combinaison avec les supports solides ou liquides acceptables en agriculture et les agents tensio-actif acceptables en agriculture.

**12.** Procédé de désherbage (notamment de zones de culture dicotylédone ou de maïs) qui consiste à appliquer aux plantes devant être détruites une quantité efficace d'un composé de formule (I) selon la revendication 1.

**13.** Procédé de désherbage (notamment de zones de culture dicotylédone ou de maïs) qui consiste à appliquer aux plantes devant être détruites une quantité efficace d'un composé de formule (I) selon la revendication 1 en pré-émergence.

**14.** Procédé de désherbage des rizières où le riz a été post repiqué par application d'une quantité efficace de 1-[(3-chloro 2-thiényl) sulfonyl] 2-[3-fluorophényl) 2-propène

**Revendications pour l'Etat contractant suivant : ES**

**1.** Composition herbicide contenant un ou plusieurs composés de formule :

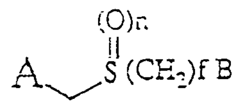

(I)

dans laquelle :

n = 0, 1, 2

f = 0, 1

A est choisi parmi les groupes

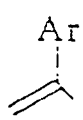

($A_1$)

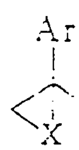

($A_2$)

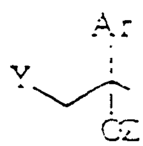

($A_3$)

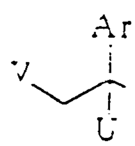

($A_4$)

dans lesquels :

Ar est choisi parmi les groupes

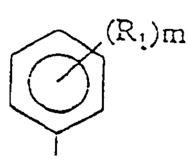

Ar-1

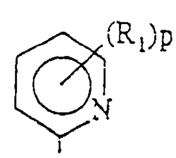

Ar-2

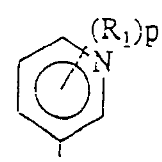

Ar-3

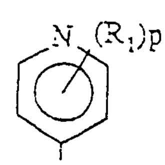

Ar-4

X étant un atome d'oxygène ou de soufre,
$R_1$ étant un atome d'hogène (notamment Cl ou Br ou F), un groupe $C_1$-$C_4$ alkyle, $C_1$-$C_4$ alkoxy, $C_1$-$C_4$ haloalkyle, $C_1$-$C_4$ haloalkoxy, nitro, cyano, $C_6$-$C_{10}$ aryle (notamment phényle ou naphtyle), $C_7$-$C_{11}$ aralkyle (notamment benzyle), $C_6$-$C_{10}$ aryloxy (notamment phénoxy ou naphthoxy) éventuellement substitué par 1 ou 2 atomes d'halogène, $C_7$-$C_{11}$ aralkyloxy (notamment benzyloxy) eventuellement substitué par 1 ou 2 atomes d'halogène,
m = 0, 1, 2, 3, 4, 5,
p = 0, 1, 2, 3, 4,
les différents radicaux $R_1$ étant identiques ou différents lorsque m ou p est supérieur ou égal à 2, Y est un atome de chlore ou de brome ou d'iode ou un groupement $OSO_2R_2$,
$R_2$ étant un groupement $C_1$-$C_8$ alkyle, $C_6$-$C_{10}$ aryle, $C_7$-$C_{11}$ aralkyle, les dits groupements étant éventuellement substitués par 1 à 3 halogènes ou groupements $C_1$-$C_4$ alkyle, $C_1$-$C_4$ alkoxy, $C_1$-$C_4$ haloalkyle, $C_1$-$C_4$ haloalkoxy, nitro, Z étant un atome d'hydrogène ou un groupe
$(C{=}O)R_5$,
$R_5$ est hydrogène, $C_1$-$C_4$ alkyle, $C_1$-$C_4$ haloalkyle,
U étant un atome de brome ou de chlore,
V étant un atome de chlore, de brome ou d'iode,
B est choisi parmi les groupes $C_2$-$C_{10}$ alcényle, ces groupes étant éventuellement substitués par 1 à 6 atomes d'halogène ou choisi parmi les groupes

B1 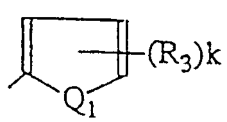 B2 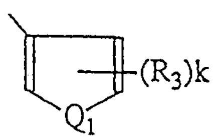

B3 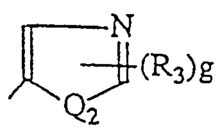 B4 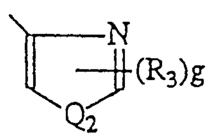 B5 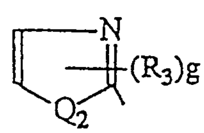

B6 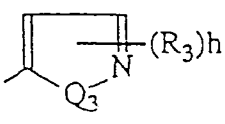 B7 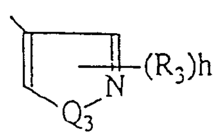 B8 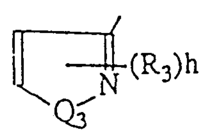

$R_3$ ayant l'une des significations indiquées pour $R_1$ ou le groupe $C_1$-$C_4$ thioalkyl, les différents radicaux $R_3$ étant identiques ou différents lorsque k ou g ou h est supérieur ou égal à 2,
$Q_1$ étant un atome de soufre, d'oxygène ou le groupe $NR_4$,
$Q_2$ étant un atome de soufre ou d'oxygène,
$Q_3$ étant un atome de soufre ou d'oxygène,
$R_4$ est hydrogène, $C_1$-$C_4$ alkyle, $C_1$-$C_4$ haloalkyle,
k = 0, 1, 2, 3,
g = 0, 1, 2,
h = 0, 1, 2.
à l'exception des composés de formule (I) où A est $A_1$ avec $A_r$ est phényle et B est 1-isopropyl 2-méthyl 1-propenyle, n = 0, f = 0 ou B est vinyle, n = 2, f = 0 ou B est 3,3,3 trifluoro 2-trifluorométhyl 1-propényle, n = 0, f = 0.

2. Composition selon la revendication 1, caractérisée en ce que n = 2.

3. Composition selon la revendication 1, caractérisée en ce que X = O.

4. Composition selon la revendication 1, caractérisée en ce que m est inférieur ou égal à 2.

5. Composition selon la revendication 1, caractérisée en ce que p est inférieur ou égal à 2.

6. Composition selon la revendication 1, caractérisée en ce que k est inférieur ou égal à 2.

7. Composition selon la revendication 1, caractérisée en ce que $R_1$ est halogène, nitro, trifluorométhyle, méthoxy, méthyle, éthyle.

8. Composition selon la revendication 1, caractérisée en ce que Z est hydrogène.

9. Composition selon la revendication 1, caractérisée en ce que U et V sont l'atome de brome.

10. Utilisation des composés selon l'une des revendications 1 à 10 à titre d'herbicide notamment sous la forme d'une composition herbicide comportant en outre un support inerte.

11. Composition herbicide comportant 0,5 à 95 % en poids d'une matière active selon l'une des revendications 1 à 10 en combinaison avec les supports solides ou liquides acceptables en agriculture et les agents tensio-actif acceptables en agriculture.

12. Procédé de désherbage (notamment de zones de culture dicotylédone ou de maïs) qui consiste à appliquer aux plantes devant être détruites une quantité efficace d'un composé de formule (I) selon la revendication 1.

13. Procédé de désherbage (notamment de zones de culture dicotylédone ou de maïs) qui consiste à appliquer aux plantes devant être détruites une quantité efficace d'un composé de formule (I) selon la revendication 1 en pré-émergence.

14. Procédé de désherbage des rizières où le riz a été post repiqué par application d'une quantité efficace de 1-[(3-chloro 2-thiényl) sulfonyl] 2-[3-fluorophényl) 2-propène.

Office européen des brevets

# RAPPORT DE RECHERCHE EUROPEENNE

Numero de la demande

EP 90 42 0573
Page 1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | EP-A-273549 (IMPERIAL CHEMICAL INDUSTRIES)<br>* page 1 *<br>--- | 1, 10 | C07D307/38<br>C07D307/64<br>C07D333/18 |
| A | EP-A-226917 (BASF AKTIENGESSELLSCHAFT)<br>* pages 1 - 2, ligne 11 *<br>--- | 1, 10 | C07D333/34<br>C07D207/333<br>C07D207/36 |
| A | EP-A-157712 (RHONE-POULENC AGROCHIMIE)<br>* pages 1 - 3, ligne 10 *<br>--- | 1, 10 | C07D263/32<br>C07D263/46<br>C07D261/08 |
| A | EP-A-107392 (PFIZER LIMITED)<br>* page 2, ligne 15 - page 3, ligne 26 *<br>--- | 1, 10 | C07D261/10<br>C07D275/03<br>C07D277/26 |
| P,A | EP-A-351332 (RHONE-POULENC AGROCHIMIE)<br>* pages 1 - 3, ligne 35 *<br>--- | 1, 10 | C07D277/36<br>C07C317/12<br>C07C317/18 |
| A | CHEMICAL ABSTRACTS, vol. 110, no. 23, 05 juin 1989<br>Columbus, Ohio, USA<br>Fujisawa Tamotsu et al.: "Diastereofacial control in the reaction of [R]-alpha-[p-tolylsulfinyl]acetophenones with several organometallics."& Chem.Lett.1988,[9], 1541-4<br>page 713; colonne 2; ref. no. 212439Q<br>* abrégé *<br>----- | 15 | C07C317/22<br>C07D401/12<br>C07D405/12<br>C07D409/12<br>C07D413/12 |

DOMAINES TECHNIQUES RECHERCHES (Int. Cl.5 )

C07C147/00
C07D307/00
C07D333/00
C07D207/00
C07D263/00
C07D261/00
C07D275/00
C07D277/00
C07D401/00
C07D405/00
C07D413/00
C07D417/00
A01N41/00
A01N43/00

Le présent rapport a été établi pour toutes les revendications

| Lieu de la recherche | Date d'achèvement de la recherche | Examinateur |
|---|---|---|
| BERLIN | 15 AVRIL 1991 | KYRIAKAKOU, G. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 90 42 0573
Page 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| | | | C07D417/12<br>C07D405/14<br>C07D409/14<br>C07F7/08<br>C07F9/40<br>A01N41/10<br>A01N43/40 |
| | | | DOMAINES TECHNIQUES RECHERCHES (Int. Cl.5) |
| | | | |

Le présent rapport a été établi pour toutes les revendications

| Lieu de la recherche | Date d'achèvement de la recherche | Examinateur |
|---|---|---|
| BERLIN | 15 AVRIL 1991 | KYRIAKAKOU, G. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)